# EUROPÄISCHE PATENTANMELDUNG

(11) **EP 0 790 242 A1**
(43) Veröffentlichungstag der Anmeldung: **20.08.1997**
(21) Anmeldenummer: 97107046.1
(22) Anmeldetag: 11.03.1988
(51) Int. Cl.: C07D 249/12, C07D 249/14, C07D 403/04, C07D 401/04, C07D 405/12, C07D 401/12, C07D 409/12, C07D 413/12, C07D 417/12, C07D 403/12, A01N 47/38

(54) **Substituierte Triazolinone**

(30) Priorität: 24.03.1987 DE 3709574
(62) Teilanmeldung aus: 93117477.5
(71) Anmelder: BAYER AG, 51368 Leverkusen (DE)
(72) Erfinder: Findeisen, Kurt, Prof.-Dr., 51375 Leverkusen (DE); Lindig, Markus, Dr., 40764 Langenfeld (DE); Santel, Hans-Joachim, Dr., 51371 Leverkusen (DE); Schmidt, Robert R., Dr., 51467 Bergisch Gladbach (DE); Lürssen, Klaus, Dr., 51469 Bergisch Gladbach (DE); Strang, Harry, Dr., 40489 Düsseldorf (DE)

(57) **Zusammenfassung**

Substituierte Triazolinone der allgemeinen Formel (IVa) 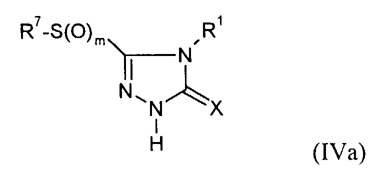 in welcher
- R¹: für Methoxy, Ethoxy, Methoxymethyl, für geradkettiges oder verzweigtes Halogenalkyl mit 1 bis 4 Kohlenstoffatomen und 1 bis 9 gleichen oder verschiedenen Halogenatomen, insbesondere Fluor, Chlor oder Brom, für Cyclopropylmethyl, Cyclohexylmethyl, Cyclohexylethyl steht,
- X: für Sauerstoff steht,
- R⁷: für Methyl, Ethyl, n-oder i-Propyl, n-, i-, s- oder t-Butyl, Allyl, Propargyl, Cyclopentyl, Cyclohexyl, Cyclohexylmethyl, Cyclohexylethyl steht und
- m: für die Zahl 0 steht,
sind Ausgangs- bzw. Zwischenprodukte für die Herstellung von 1-substituierten Triazolionen mit herbizider und pflanzenwachstumsproduktorischer Wirkung.

## Beschreibung

Die Erfindung betrifft neue substituierte Triazolinone, mehrere Verfahren zu ihrer Herstellung und ihre Verwendung als Herbizide und Pflanzenwachstumsregulatoren.

Es ist bekannt, daß bestimmte Stickstoffheterocyclen wie beispielsweise das Imidazolidin-2-on-1-carbonsäureisobutylamid (vgl. z.B. K.H. Büchel ,,Pflanzenschutz und Schädlingsbekämpfung" S. 170, Thieme Verlag Stuttgart 1977) oder das 1-Phenyl-3-(3-trifluormethylphenyl)-5-methyl-perhydropyrimidin-2-on (vgl. z.B. EP-A-58 868 oder DE-A-3 237 479) herbizide Eigenschaften besitzen.

Die herbizide Wirksamkeit dieser vorbekannten Verbindungen gegenüber Problemunkräutern ist jedoch ebenso wie ihre Verträglichkeit gegenüber wichtigen Kulturpflanzen nicht in allen Anwendungsgebieten völlig zufriedenstellend.

Weiterhin sind bestimmte substituierte Triazolinone, wie beispielsweise das 1-(N,N-Dimethylcarbamoyl)-3-isopropylthio-4-methyl-1,2,4-triazolin-5-thion oder das 1-(N,N-Dimethylcarbamoyl)-3-ethylthio-4-methyl-1,2,4-triazolin-5-thion oder das 1-(N,N-Dimethylcarbamoyl)-3-isopropylthio-4-methyl-1,2,4-triazolin-5-on oder das 1-(N,N-Dimethylcarbamoyl)-3-ethylthio-4-methyl-1,2,4-triazolin-5-on oder das 1-(N,N-Dimethylcarbamoyl)-3-methylthio-4-methyl-1,2,4-triazolin-5-on oder das 1-(N,N-Dimethylcarbamoyl)-3-propylthio-4-methyl-1,2,4-triazolin-5-thion oder das 1-(N,N-Dimethylcarbamoyl)-3-allylthio-4-methyl-1,2,4-triazolin-5-thion oder das 1-(N,N-Dimethylcarbamoyl)-3-methylthio-4-methyl-1,2,4-triazolin-5-thion bekannt (vgl. DE-A-2 707 801). Über eine Wirksamkeit dieser vorbekannten Triazolinone als Herbizide oder Pflanzenwachstumsregulatoren ist bisher nichts bekannt.

Weitere Triazolinone werden in Tetrahedron 22, 1999-2006 (1977) beschrieben und hier insbesondere die Verbindungen 2(H)-Carbamoyl-3-hydroxy-5-methylthio-1,2,4-triazol, 5-Benzylthio-2(H)-carbamoyl-3-hydroxy-1,2,4-triazol und 5-Benzylthio-3-hydroxy-2(H)-phenylcarbamoyl-1,2,4-triazol.

Außerdem werden Triazolinone, jedoch meist mit anderem Substitutionsmuster am Triazolring in folgenden Druckschriften offenbart
- DE-A-2 042 660: unter Hinweis auf pflanzenschützende Eigenschaften
- US-A-3 308 131: unter Hinweis auf breite Verwendungsmöglichkeiten wie Herbizide, Gummiadditive etc.,
- JP-A 53 135 981: und
- JP-A 52 125 168: beide mit Hinweisen auf herbizide Verwendbarkeit.

Schließlich seien noch die Patentschriften EP-A-294 666, EP-A-298 371 und EP-A-305 844 genannt, die zum Zeitpunkt der Anmeldung der vorliegenden Patentanmeldung nicht veröffentlicht waren, jedoch alle 1,2,4-trisubstituierte Triazolinone mit herbiziden Eigenschaften offenbaren.

Es wurden neue substituierte Triazolinone der allgemeinen Formel (I), 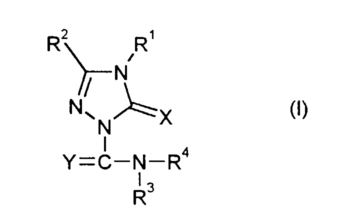 in welcher
- R¹: für Methyl, Ethyl, n- oder i-Propyl, n-, i-, s- oder t-Butyl, n- oder i-Pentyl, n- oder i-Hexyl, für Allyl, Propargyl, Methoxy, Ethoxy, Methoxymethyl, für geradkettiges oder verzweigtes Halogenalkyl mit 1 bis 4 Kohlenstoffatomen und 1 bis 9 gleichen oder verschiedenen Halogenatomen, insbesondere Fluor, Chlor oder Brom, für Cyclopentyl, Cyclohexyl, Cyclopropyl, Cyclopropylmethyl, Cyclohexylmethyl, Cyclohexylethyl oder für jeweils gegebenenfalls ein- bis dreifach gleich oder verschieden durch Fluor, Chlor, Brom, Cyano, Nitro, Methyl, Ethyl, n- oder i-Propyl, n-, i-, s- oder t-Butyl, Methoxy, Ethoxy, Methylthio, Trifluormethyl, Trifluormethoxy oder Trifluormethylthio substituiertes Benzyl oder Phenyl steht,
- R²: für einen Rest 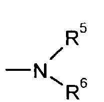 oder für einen Rest S(O)ₙ-R⁷ steht
- R³ und R⁴: unabhängig voneinander jeweils für Wasserstoff, Methyl, Ethyl, n- oder i- Propyl, n-, i-, s- oder t-Butyl, n- oder i-Pentyl, n- oder i-Hexyl, n- oder i-Heptyl, n- oder i-Octyl, n- oder i-Nonyl, n- oder i-Decyl, n- oder i-Dodecyl, für Allyl, n- oder i-Butenyl, n- oder i-Pentenyl, n- oder i-Hexenyl, Propargyl, n- oder i-Butinyl, n- oder i-Pentinyl, n- oder i-Hexinyl, für geradkettiges oder verzweigtes Halogenalkyl mit 1 bis 6 Kohlenstoffatomen und 1 bis 9 gleichen oder verschiedenen Halogenatomen, insbesondere Fluor, Chlor oder Brom für jeweils geradkettiges oder verzweigtes Halogenalkenyl oder Halogenalkinyl mit jeweils 3 bis 5 Kohlenstoffatomen und 1 bis 3 Halogenatomen, insbesondere Fluor oder Chlor, für jeweils geradkettiges oder verzweigtes Cyanalkyl mit 1 bis 6 Kohlenstoffatomen im Alkylteil, Hydroxyalkyl mit 1 bis 6 Kohlenstoffatomen und 1 bis 3 Hydroxygruppen, Alkoxyalkyl, Alkoxycarbonylalkyl oder Alkoxycarbonylalkenyl, Alkylaminoalkyl oder Dialkylaminoalkyl mit jeweils bis zu 4 Kohlenstoffatomen in den einzelnen Alkyl- und Alkenylteilen oder für jeweils gegebenenfalls ein- bis dreifach, gleich oder verschieden durch Fluor, Chlor, Brom, Methyl, Ethyl, n- oder i-Propyl, n-, i-, s- oder t-Butyl, Cyano, Methandiyl, Ethandiyl, Butandiyl oder Butandiendiyl substituiertes Cyclopropyl, Cyclopropylmethyl, Cyclopropylethyl, Cyclopentyl, Cyclohexyl, Cycloheptyl, Cyclooctyl, Cyclohexylmethyl, Cyclohexylethyl, Cyclohexenyl oder Cyclohexenylmethyl stehen; außerdem für im Heterocyclylteil gegebenenfalls ein- bis dreifach, gleich oder verschieden durch Fluor, Chlor, Brom, Cyano, Nitro, Methyl, Ethyl, n- oder i-Propyl, n-, i-, s- oder t-Butyl, Methoxy, Ethoxy, Methylthio, Trifluormethyl, Trifluormethoxy oder Trifluormethylthio substituiertes Heterocyclylmethyl, Heterocyclylpropyl oder Heterocyclylethyl mit den Heterocyclen 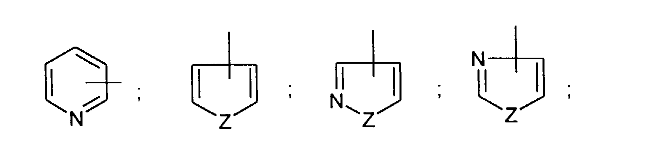 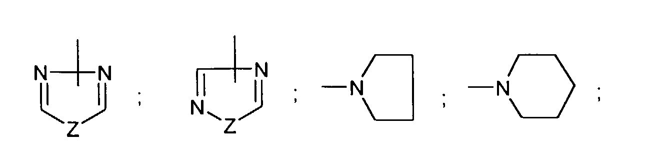 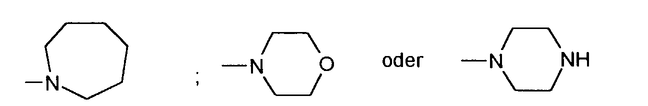 stehen,
wobei Z jeweils für Sauerstoff oder Schwefel steht,
außerdem für jeweils geradkettiges oder verzweigtes Alkoxy mit 1 bis 6 Kohlenstoffatomen, Alkenyloxy mit 3 bis 6 Kohlenstoffatomen oder Alkinyloxy mit 3 bis 6 Kohlenstoffatomen oder für jeweils gegebenenfalls im Phenylteil ein- bis dreifach, gleich oder verschieden durch Fluor, Chlor, Brom, Hydroxy, Cyano, Nitro, Methyl, Ethyl, n- oder i-Propyl, n-, i-, s- oder t-Butyl, Methoxy, Ethoxy, Methylthio, Trifluormethyl, Trifluormethoxy, Trifluormethylthio, Trifluormethylsulfinyl, Trifluormethylsulfonyl, Methylsulfinyl, Methylsulfonyl, Acetyl, Propionyl, Methoxycarbonyl, Ethoxycarbonyl, Cyclohexyl oder Phenoxy substituiertes, gegebenenfalls geradkettiges oder verzweigtes Benzyl, Phenylethyl, Phenylpropyl, Phenylbutyl, Phenylpentyl, Phenylhexyl, Phenylheptyl, Phenylcyanmethyl, Phenylcyanethyl, Phenylcyanpropyl, Benzyloxy, Phenylethyloxy, Phenoxy, Benzoyl, Phenyl oder Naphthyl stehen, oder
- R³ und R⁴: gemeinsam mit dem Stickstoffatom, an welches sie gebunden sind, für einen gegebenenfalls ein- bis dreifach, gleich oder verschieden durch Methyl, Ethyl, n- oder i-Propyl, Chlor oder Trifluormethyl, substituierten Heterocyclus der Formel 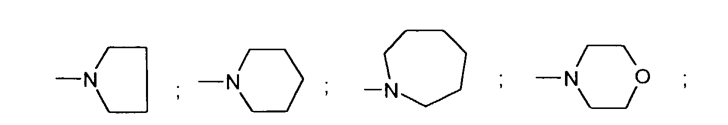 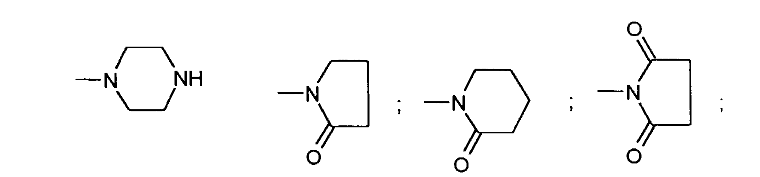 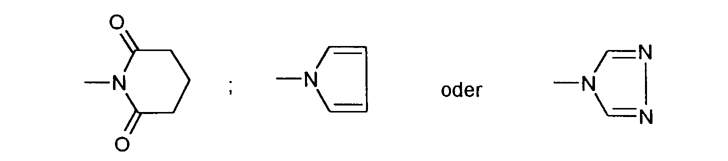 stehen,
- X: für Sauerstoff oder Schwefel steht und
- Y: für Sauerstoff oder Schwefel steht, wobei
- R⁵ und R⁶: unabhängig voneinander jeweils für Methyl, Ethyl, n- oder i-Propyl, n-, i-, s- oder t-Butyl, n- oder i-Pentyl, Allyl, Propargyl, für jeweils geradkettiges oder verzweigtes Halogenalkyl mit 1 bis 4 Kohlenstoffatomen, Halogenalkenyl mit 3 bis 6 Kohlenstoffatomen oder Halogenalkinyl mit 3 bis 6 Kohlenstoffatomen und jeweils 1 bis 9 gleichen oder verschiedenen Halogenatomen, für Methoxymethyl, Methoxyethyl, Methoxy, Ethoxy, für Cyclopropyl, Cyclopropylmethyl, Cyclopentyl, Cyclohexyl, Cyclohexylmethyl, Cyclohexylethyl, Cyclopentylmethyl oder für jeweils gegebenenfalls ein- bis dreifach, gleich oder verschieden durch Fluor, Chlor, Brom, Cyano, Nitro, Methyl, Ethyl, n- oder i-Propyl, n-, i-, s- oder t-Butyl, Methoxy, Ethoxy, Methylthio, Trifluormethyl, Trifluormethoxy oder Trifluormethylthio substituiertes Benzyl, Phenylethyl oder Phenyl stehen, oder
- R⁵ und R⁶: gemeinsam mit dem Stickstoffatom, an welches sie gebunden sind, für einen gegebenenfalls ein- bis dreifach, gleich oder verschieden durch Methyl, Ethyl, n- oder i-Propyl, Chlor oder Trifluormethyl, substituierten Heterocyclus der Formel 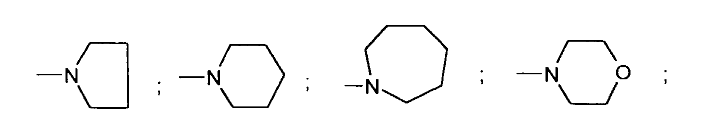 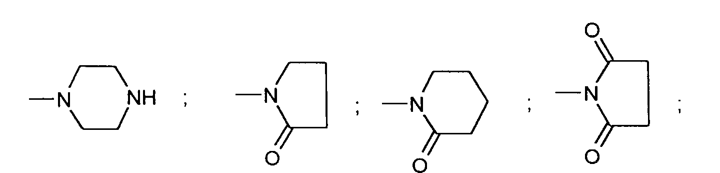 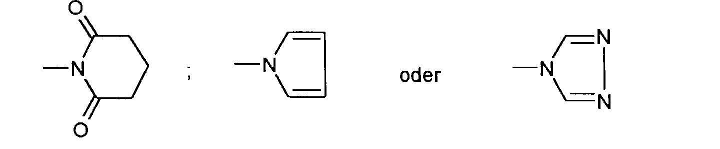 stehen,
- R⁷: für Methyl, Ethyl, n- oder i-Propyl, n-, i-, s- oder t-Butyl, Allyl, Propargyl, Cyclopentyl, Cyclohexyl, Cyclohexylmethyl, Cyclohexylethyl oder für jeweils gegebenenfalls ein- bis dreifach, gleich oder verschieden durch Fluor, Chlor, Brom, Cyano, Nitro, Methyl, Ethyl, n- oder i-Propyl, n-, i-, s- oder t-Butyl, Methoxy, Ethoxy oder Trifluormethyl substituiertes Benzyl oder Phenyl steht, und
- n: für eine Zahl 0, 1 oder 2 steht,
wobei jedoch R² nur dann für einen Rest -S(O)ₙ-R⁷ steht, wenn R³ und R⁴ nicht gleichzeitig für Methyl stehen, ausgenommen die Verbindungen 5-Benzylthio-3-hydroxy-2(H)-phenylcarbamoyl-1,2,4-triazol, 2(H)-Carbamoyl-3-hydroxy-5-methylthio-1,2,4-triazol und 5-Benzylthio-2(H)-carbamoyl-3-hydroxy-1,2,4-triazol.

Weiterhin wurde gefunden, daß man die neuen substituierten Triazolinone der allgemeinen Formel (I), 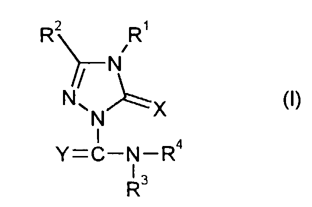 in welcher
R¹, R², R³, R⁴, X und Y die oben angegebene Bedeutung haben,
erhält, wenn man
a) 1-Chlor-(thio)-carbonyltriazolinone der Formel (II), 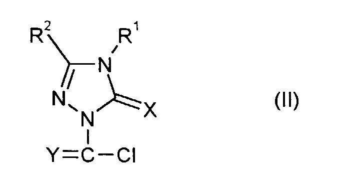 in welcher
   R¹, R², X und Y die oben angegebene Bedeutung haben,
   mit Aminen der Formel (III), 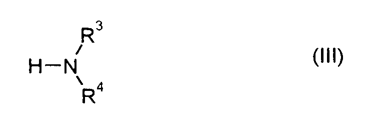 in welcher
   R³ und R⁴ die oben angegebene Bedeutung haben,
   gegebenenfalls in Gegenwart eines Verdünnungsmittels und gegebenenfalls in Gegenwart eines Säurebindemittels umsetzt, oder
b) für den Fall, daß R³ Wasserstoff bedeutet, wenn man in 1-Stellung unsubstituierte Triazolinone der Formel (IV) 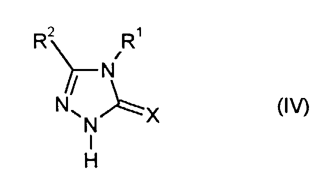 in welcher
   R¹, R² und X die oben angegebene Bedeutung haben,
   mit Iso(thio)cyanaten der Formel (V),

   R⁴-N=C=Y (V)

   in welcher
   R⁴ und Y die oben angegebene Bedeutung haben,
   gegebenenfalls in Gegenwart eines Verdünnungsmittels und gegebenenfalls in Gegenwart eines Reaktionshilfsmittels umsetzt.

Schließlich wurde gefunden, daß die neuen substituierten Triazolinone der allgemeinen Formel (I) herbizide und wachstumsregulatorische Eigenschaften besitzen.

Überraschenderweise zeigen die erfindungsgemäßen substituierten Triazolinone der allgemeinen Formel (I) eine erheblich höhere herbizide Potenz gegenüber Problemunkräutern, als die aus dem Stand der Technik bekannten Stickstoffheterocyclen, wie beispielsweise das Imidazolin-2-on-1-carbonsäureisobutylamid oder das 1-Phenyl-3-(3-trifluormethylphenyl)-5-methyl-perhydropyrimidin-2-on, welches chemisch und wirkungsmäßig naheliegende Verbindungen sind und darüber hinaus zusätzlich auch wachstumsregulatorische Eigenschaften.

Im einzelnen sei auf die bei den Herstellungsbeispielen genannten Verbindungen verwiesen.

Verwendet man beispielsweise 1-Chlorcarbonyl-3-dimethylamino-4-methyl-1,2,4-triazolin-5-on und Allylamin als Ausgangsstoffe, so läßt sich der Reaktionsablauf des erfindungsgemäßen Verfahrens (a) durch das folgende Formelschema darstellen:

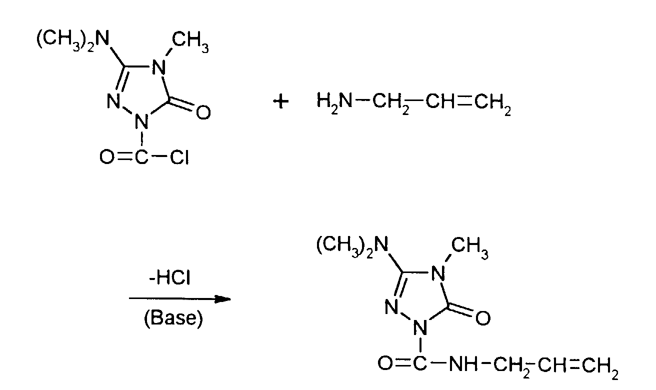

Verwendet man beispielsweise 3-Dimethylamino-4-methyl-1H-1,2,4-triazolin-5-on und Isopropylisocyanat als Ausgangsstoffe, so läßt sich der Reaktionsablauf des erfindungsgemäßen Verfahrens (b) durch das folgende Formelschema darstellen:

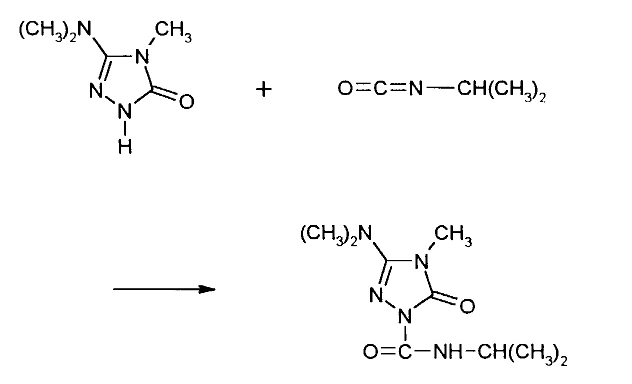

Die zur Durchführung des erfindungsgemäßen Verfahrens (a) als Ausgangsstoffe benötigten Chlor(thio)carbonyltriazolinone sind durch die Formel (II) allgemein definiert. In dieser Formel (II) stehen R¹, R², X und Y vorzugsweise für diejenigen Reste, die bereits im Zusammenhang mit der Beschreibung der erfindungsgemäßen Stoffe der Formel (I) als bevorzugt für diese Substituenten genannt wurden.

Die Chlor(thio)carbonyltriazolinone der Formel (II) sind noch nicht bekannt. Man erhält sie, wenn man in 1-Stellung unsubstituierte Triazolinone der Formel (IV), 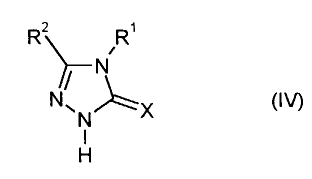 in welcher
R¹, R² und X die oben angegebene Bedeutung haben,
mit (Thio)Phosgen der Formel (VI), 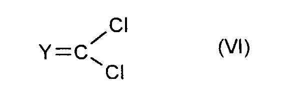 in welcher
- Y: die oben angegebene Bedeutung hat,
gegebenenfalls in Gegenwart eines Verdünnungsmittels wie beispielsweise Toluol oder Acetonitril und gegebenenfalls in Gegenwart eines Säurebindemittels wie beispielsweise Triethylamin bei Temperaturen zwischen +20°C und +150°C umsetzt,
Chlor(thio)carbonylverbindungen der Formel (IIa), 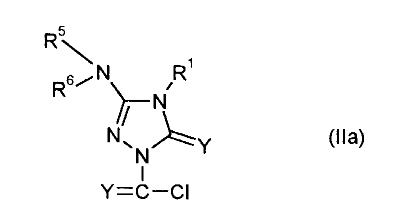 in welcher
R¹, R⁵, R⁶ und Y die oben angegebene Bedeutung haben,
erhält man alternativ auch, wenn man Aminoguanidiniumhydrochloride der Formel (VII), 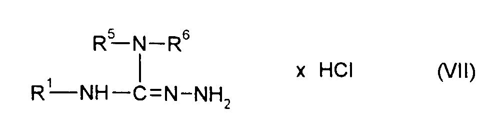 in welcher
R¹, R⁵ und R⁶ die oben angegebene Bedeutung haben,
mit doppelt-molarem Überschuß an (Thio)Phosgen der Formel (VI), 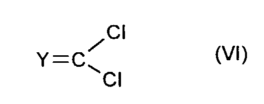 in welcher
- Y: die oben angegebene Bedeutung hat,
gegebenenfalls in Gegenwart eines Verdünnungsmittels wie beispielsweise Toluol oder Acetonitril und gegebenenfalls in Gegenwart eines Säurebindemittels bei Temperaturen zwischen +20°C und +150°C umsetzt.

Die Aminoguanidiniumhydrochloride der Formel (VII) erhält man in Analogie zu bekannten Verfahren, beispielsweise wenn man die allgemein bekannten Harnstoffe der Formel (VIII), 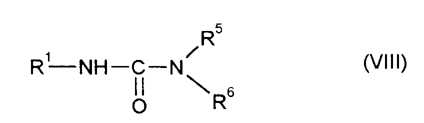 in welcher
R¹, R⁵ und R⁶ die oben angegebene Bedeutung haben,
zunächst in einer 1. Stufe mit (Thio)Phosgen der Formel (VI), 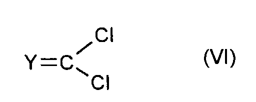 in welcher
- Y: die oben angegebene Bedeutung hat,
gegebenenfalls in Gegenwart eines Verdünnungsmittels wie beispielsweise Toluol oder Acetonitril bei Temperaturen zwischen +10°C und +150°C umsetzt, die so erhältlichen Formamidinhydrochloride der Formel (IX), 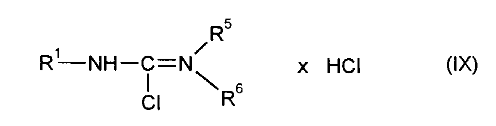 in welcher
R¹, R⁵ und R⁶ die oben angegebene Bedeutung haben,
in einer 2. Stufe mit Hydrazinhydrat, gegebenenfalls in Gegenwart eines Verdünnungsmittels wie beispielsweise Isopropanol oder Dichlormethan bei Temperaturen zwischen -10°C und +60°C umsetzt (vgl. z.B. J. org. Chem. 19, 1807 (1954); Bull. Soc. Chim. Fr. 1975, 1649; US 2 845 458).

Harnstoffe der Formel (VIII), Phosgen und Thiophosgen der Formel (VI) sind allgemein bekannte Verbindungen der organischen Chemie.

Die zur Durchführung des erfindungsgemäßen Verfahrens (a) weiterhin als Ausgangsstoffe benötigten Amine sind durch die Formel (III) allgemein definiert. In dieser Formel (III) stehen R³ und R⁴ vorzugsweise für diejenigen Reste, die bereits im Zusammenhang mit der Beschreibung der erfindungsgemäßen Stoffe der Formel (I) als bevorzugt für diese Substituenten genannt wurden.

Die Amine der Formel (III) sind allgemein bekannte Verbindungen der organischen Chemie.

Die zur Durchführung des erfindungsgemäßen Verfahrens (b) und zur Synthese der Vorprodukte der Formel (II) als Ausgangsstoffe benötigten in 1-Stellung unsubstituierten Triazolinone sind durch die Formel (IV) allgemein definiert. In dieser Formel (IV) stehen R¹, R², und X vorzugsweise für diejenigen Reste, die bereits im Zusammenhang mit der Beschreibung der erfindungsgemäßen Stoffe der Formel (I) als bevorzugt für diese Substituenten genannt wurden.

Die in 1-Stellung unsubstituierten Triazolinone der Formel (IV) sind teilweise bekannt (vgl. z.B. Chem. Ber. 102, 735 (1969); Chem. Ber. 107, 454 (1974); Arch. Pharm. 307, 509 (1974); Helv. Chem. Acta 63, 841 (1980); US 4098 896; US 4 110 332; US 4 530 898; DE-OS 2 250 572; J. chem. Soc. C. 1967, 746; J. Chem. Soc. Perkin Trans. I, 1059 (1982); Arzneimittel Forsch. 27, 343 (1977); Compt. Rend. 253, 174 (1961); Bull. Soc. Chim. Fr. 1963, 144; French Patent FR M 1559 vom 3.12.62). Man erhält die bekannten, ebenso wie die nichtbekannten Verbindungen der Formel (IV) in Analogie zu bekannten Verfahren (vgl. z.B. J. org. Chem. 51, 1719 (1986); US 4098 896 sowie die Herstellungsbeispiele). In 1-Stellung unsubstituierte Triazolinone der Formel (IVa) 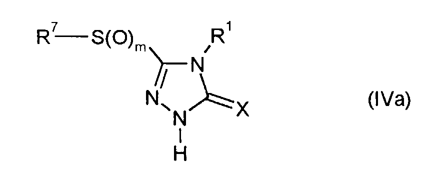 in welcher
R¹, R⁷ und X die oben angegebene Bedeutung haben und
- m: für eine Zahl 1 oder 2 steht,
erhält man aus den entsprechenden Verbindungen der Formel (IVb), 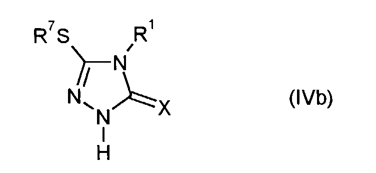 in welcher
R¹, R⁷ und X die oben angegebene Bedeutung haben,
in allgemein bekannter Art und Weise mit üblichen Oxidationsmitteln, beispielsweise durch Umsetzung mit 3-Chlorperbenzoesäure gegebenenfalls in Gegenwart eines Verdünnungsmittels wie beispielsweise Dichlormethan oder Acetonitril und gegebenenfalls in Gegenwart eines Katalysators wie beispielsweise Ammoniummolybdat bei Temperaturen zwischen 0°C und 40°C.

Die zur Durchführung des erfindungsgemäßen Verfahrens (b) weiterhin als Ausgangsstoffe benötigten Iso(thio)-cyanate sind durch die Formel (V) allgemein definiert. In dieser Formel (V) stehen R⁴ und Y vorzugsweise für diejenigen Reste, die bereits im Zusammenhang mit der Beschreibung der erfindungsgemäßen Stoffe der Formel (I) als bevorzugt für diese Substituenten genannt wurden.

Die Iso(thio)cyanate sind allgemein bekannte Verbindungen der organischen Chemie (vgl. z.B. Saul Patai, ,,The Chemistry of Cyanates and their Thioderivates" J. Wiley & Sons, New York 1977).

Als Verdünnungsmittel zur Durchführung des erfindungsgemäßen Verfahrens (a) kommen vorzugsweise inerte organische Lösungsmittel in Frage.

Hierzu gehören insbesondere aliphatische, alicyclische oder aromatische, gegebenenfalls halogenierte Kohlenwasserstoffe, wie beispielsweise Benzin, Ligroin, Benzol, Toluol, Xylol, Chlorbenzol, Petrolether, Pentan, Hexan, Heptan, Cyclohexan, Dichlormethan, Chloroform, Tetrachlorkohlenstoff, Ether, wie Diethylether, Dioxan, Tetrahydrofuran oder Ethylenglykoldimethyl oder -diethylether, Ketone wie Aceton oder Butanon, Nitrile, wie Acetonitril oder Propionitril, Amide, wie Dimethylformamid, Dimethylacetamid, N-Methylformanilid, N-Methylpyrrolidon oder Hexamethylphosphorsäuretriamid, Ester, wie Essigsäureethylester oder Basen wie Pyridin.

Das erfindungsgemäße Verfahren (a) wird gegebenenfalls in Gegenwart eines geeigneten Säurebindemittels durchgeführt.

Als solche kommen alle üblichen anorganischen oder organischen Basen in Frage. Hierzu gehören beispielsweise Alkalimetallhydroxide, wie Natriumhydroxid oder Kaliumhydroxid, Alkalimetallcarbonate, wie Natriumcarbonat, Kaliumcarbonat oder Natriumhydrogencarbonat, sowie tertiäre Amine, wie Triethylamin, N,N-Dimethylanilin, Pyridin, N,N-Dimethylaminopyridin, Diazabicyclooctan (DABCO), Diazabicyclononen (DBN) oder Diazabicycloundecen (DBU).

Es ist auch möglich, das als Reaktionspartner verwendete Amin der Formel (III) in entsprechendem Überschuß gleichzeitig als Säurebindemittel einzusetzen.

Die Reaktionstemperaturen können bei der Durchführung des erfindungsgemäßen Verfahrens (a) in einem größeren Bereich variiert werden. Im allgemeinen arbeitet man bei Temperaturen zwischen 0°C und +150°C, vorzugsweise bei Temperaturen zwischen +10°C und +80°C.

Das erfindungsgemäße Verfahren (a) wird üblicherweise unter Normaldruck durchgeführt. Es ist jedoch auch möglich unter erhöhtem Druck zu arbeiten.

Zur Durchführung des erfindungsgemäßen Verfahrens (a) setzt man pro Mol an 1-Chlor-(thio)carbonyl-triazolinon der Formel (II) im allgemeinen 1,0 bis 5,0 mol, vorzugsweise 1,0 bis 2,5 mol an Amin der Formel (III) und gegebenenfalls 1,0 bis 2,5 mol an Säurebindemittel ein. Die Reaktionsdurchführung, Aufarbeitung und Isolierung der Reaktionsprodukte erfolgt in Analogie zu allgemein bekannten Verfahren.

Als Verdünnungsmittel zur Durchführung des erfindungsgemäßen Verfahrens (b) kommen ebenfalls inerte organische Lösungsmittel in Frage. Vorzugsweise verwendet man die bei Verfahren (a) genannten Verdünnungsmittel.

Das erfindungsgemäße Verfahren (b) kann gegebenenfalls in Gegenwart eines basischen Reaktionshilfsmittels durchgeführt werden. Als solche kommen alle üblichen anorganischen und organischen Basen in Frage. Vorzugsweise verwendet man tertiäre Amine, wie Triethylamin, N,N-Dimethylanilin, Pyridin, N,N-Dimethylaminopyridin, Diazabicyclooctan (DABCO), Diazabicyclononen (DBN) oder Diazabicycloundecen (DBU).

Der Zusatz solcher Katalysatoren ist jedoch nicht zwingend erforderlich.

Die Reaktionstemperaturen können bei der Durchführung des erfindungsgemäßen Verfahrens (b) in einem größeren Bereich variiert werden. Im allgemeinen arbeitet man bei Temperaturen zwischen 0°C und +150°C, vorzugsweise bei Temperaturen zwischen +40°C und +120°C.

Das erfindungsgemäße Verfahren (b) wird üblicherweise unter Normaldruck durchgeführt. Es ist jedoch auch möglich, insbesondere bei gasförmigen Ausgangsverbindungen, unter erhöhtem Druck zu arbeiten.

Zur Durchführung des erfindungsgemäßen Verfahrens (b) setzt man pro Mol an in 1-Stellung unsubstituiertem Triazolinon der Formel (IV) im allgemeinen 1,0 bis 5,0 mol, vorzugsweise 1,0 bis 2,5 mol, an Iso(thio)cyanat der Formel (V) und gegebenenfalls 1,0 bis 2,5 mol an Reaktionshilfsmittel ein. Die Reaktionsdurchführung, Aufarbeitung und Isolierung der Reaktionsprodukte erfolgt in Analogie zu allgemein bekannten Verfahren.

Die erfindungsgemäßen Wirkstoffe können als Defoliants, Desiccants, Krautabtötungsmittel und insbesondere als Unkrautvernichtungsmittel verwendet werden. Unter Unkraut im weitesten Sinne sind alle Pflanzen zu verstehen, die an Orten aufwachsen, wo sie unerwünscht sind. Ob die erfindungsgemäßen Stoffe als totale oder selektive Herbizide wirken, hängt im wesentlichen von der angewendeten Menge ab.

Die erfindungsgemäßen Wirkstoffe können z.B. bei den folgenden Pflanzen verwendet werden:

Dikotyle Unkräuter der Gattungen: Sinapis, Lepidium, Galium, Stellaria, Matricaria, Anthemis, Galinsoga, Chenopodium, Urtica, Senecio, Amaranthus, Portulaca, Xanthium, Convolvulus, Ipomoea, Polygonum, Sesbania, Ambrosia, Cirsium, Carduus, Sonchus, Solanum, Rorippa, Rotala, Lindernia, Lamium, Veronica, Abutilon, Emex, Datura, Viola, Galeopsis, Papaver, Centaurea, Trifolium, Ranunculus, Taraxacum.

Dikotyle Kulturen der Gattungen: Gossypium, Glycine, Beta, Daucus, Phaseolus, Pisum, Solanum, Linum, Ipomoea, Vicia, Nicotiana, Lycopersicon, Arachis, Brassica, Lactuca, Cucumis, Cucurbita.

Monokotyle Unkräuter der Gattungen: Echinochloa, Setaria, Panicum, Digitaria, Phleum, Poa, Festuca, Eleusine, Brachiaria, Lolium, Bromus, Avena, Cyperus, Sorghum, Agropyron, Cynodon, Monochoria, Fimbristylis, Sagittaria, Eleocharis, Scirpus, Paspalum, Ischaemum, Sphenoclea, Dactyloctenium, Agrostis, Alopecurus, Apera.

Monokotyle Kulturen der Gattungen: Oryza, Zea, Triticum, Hordeum, Avena, Secale, Sorghum, Panicum, Saccharum, Ananas, Asparagus, Allium.

Die Verwendung der erfindungsgemäßen Wirkstoffe ist jedoch keineswegs auf diese Gattungen beschränkt, sondern erstreckt sich in gleicher Weise auch auf andere Pflanzen.

Die Verbindungen eignen sich in Abhängigkeit von der Konzentration zur Totalunkrautbekämpfung z.B. auf Industrie- und Gleisanlagen und auf Wegen und Plätzen mit und ohne Baumbewuchs. Ebenso können die Verbindungen zur Unkrautbekämpfung in Dauerkulturen, z.B. Forst, Ziergehölz-, Obst-, Wein-, Citrus-, Nuß-, Bananen-, Kaffee-, Tee-, Gummi-, Ölpalm-, Kakao-, Beerenfrucht- und Hopfenanlagen, auf Zier- und Sportrasen und Weideflächen und zur selektiven Unkrautbekämpfung in einjährigen Kulturen eingesetzt werden.

Dabei lassen sich die erfindungsgemäßen Wirkstoffe mit besonders gutem Erfolg zur Bekämpfung mono- und dikotyler Unkräuter insbesondere in monokotylen Kulturen einsetzen.

Darüber hinaus greifen die erfindungsgemäßen Wirkstoffe in den Metabolismus der Pflanzen ein und können deshalb als Wachstumsregulatoren eingesetzt werden.

Für die Wirkungsweise von Pflanzenwachstumsregulatoren gilt nach der bisherigen Erfahrung, daß ein Wirkstoff auch mehrere verschiedenartige Wirkungen auf Pflanzen ausüben kann. Die Wirkungen der Stoffe hängen im wesentlichen ab von dem Zeitpunkt der Anwendung bezogen auf das Entwicklungsstadium der Pflanze sowie von den auf die Pflanzen oder ihre Umgebung ausgebrachten Wirkstoffmengen und von der Art der Applikation. In jedem Fall sollen Wachstumsregulatoren die Kulturpflanzen in bestimmter gewünschter Weise beeinflussen.

Unter dem Einfluß von Wachstumsregulatoren kann der Blattbestand der Pflanzen so gesteuert werden, daß ein Entblättern der Pflanzen zu einem gewünschten Zeitpunkt erreicht wird. Eine derartige Entlaubung spielt bei der mechanischen Beerntung der Baumwolle eine große Rolle, ist aber auch in anderen Kulturen, wie z.B. im Weinbau, zur Erleichterung der Ernte von Interesse. Eine Entlaubung der Pflanzen kann auch vorgenommen werden, um die Transpiration der Pflanzen vor dem Verpflanzen herabzusetzen.

In entsprechenden Aufwandmengen zeigen die erfindungsgemäßen Wirkstoffe außerdem auch insektizide, bakterizide und fungizide Wirksamkeit und lassen sich beispielsweise zur Bekämpfung von Hygiene und Vorratsschädlingen oder zur Bekämpfung von Pilzkrankheiten im Getreide- und Reisanbau, wie beispielsweise gegen den Erreger des Getreidemehltaus (Erysiphe graminis) oder gegen den Erreger der Reisfleckenkrankheit (Pyricularia oryzae) einsetzen. In diesem Anwendungsbereich zeigen die erfindungsgemäßen Wirkstoffe neben guten protektiven auch systemische Eigenschaften.

Die Wirkstoffe können in Abhängigkeit von ihren jeweiligen physikalischen und/oder chemischen Eigenschaften in übliche Formulierungen übergeführt werden, wie Lösungen, Emulsionen, Suspensionen, Pulver, Schäume, Pasten, Granulate, Aerosole, Wirkstoff-imprägnierte Natur- und synthetische Stoffe, Feinstverkapselungen in polymeren Stoffen und in Hüllmassen für Saatgut, ferner in Formulierungen mit Brennsätzen, wie Räucherpatronen, -dosen, -spiralen u.ä., sowie ULV-Kalt- und Warmnebel-Formulierungen.

Diese Formulierungen werden in bekannter Weise hergestellt, z.B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten oder chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, Alkohole, wie Butanol oder Glycol sowie deren Ether und Ester, Ketone, wie Aceton, Methylethylketon, Methylisobutylketon oder Cyclohexanon, stark polare Lösungsmittel, wie Dimethylformamid und Dimethylsulfoxid, sowie Wasser; mit verflüssigten gasförmigen Streckmitteln oder Trägerstoffen sind solche Flüssigkeiten gemeint, welche bei normaler Temperatur und unter Normaldruck gasförmig sind, z.B. Aerosol-Treibgas, wie Halogenkohlenwasserstoffe sowie Butan, Propan, Stickstoff und Kohlendioxid; als feste Trägerstoffe kommen in Frage: z.B. natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate; als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengel; als Emulgier und/oder schaumerzeugende Mittel kommen in Frage: z.B. nichtionogene und anionische Emulgatoren, wie Polyoxyethylen-Fettsäure-Ester, Polyoxyethylen-Fettalkohol-Ether, z.B. Alkylarylpolyglykol-Ether, Alkylsulfonate, Alkylsulfate, Arylsulfonate sowie Eiweißhydrolysate; als Dispergiermittel kommen in Frage: z.B. Lignin-Sulfitablaugen und Methylcellulose.

Es können in den Formulierungen Haftmittel wie Carboxy-methylcellulose, natürliche und synthetische pulverige, körnige oder latexförmige Polymere verwendet werden, wie Gummiarabicum, Polyvinylalkohol, Polyvinylacetat, sowie natürliche Phospholipide, wie Kephaline und Lecithine, und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die erfindungsgemäßen Wirkstoffe können als solche oder in ihren Formulierungen auch in Mischung mit bekannten Herbiziden zur Unkrautbekämpfung Verwendung finden, wobei Fertigformulierungen oder Tankmischungen möglich sind.

Für die Mischungen kommen bekannte Herbizide wie z.B. 1-Amino-6-ethylthio-3-(2,2-dimethylpropyl)-1,3,5-triazin-2,4(1H,3H)-dion oder N-(2-Benzthiazolyl)-N,N'-dimethylharnstoff zur Unkrautbekämpfung in Getreide; 4-Amino-3-methyl-6-phenyl-1,2,4-triazin-5(4H)-on zur Unkrautbekämpfung in Zuckerrüben und 4-Amino-6-(1,1-dimethylethyl)-3-methylthio-1,2,4-triazin-5(4H)-on zur Unkrautbekämpfung in Sojabohnen, in Frage. Auch Mischungen mit N,N-Dimethyl-N'-(3-chlor-4-methylphenyl)-harnstoff;
N,N-Dimethyl-N'-(4-isopropylphenyl)-harnstoff;
2-Chlor-4-ethylamino-6-isopropylamino-1,3,5-triazin;
4-Ethylamino-2-t-butylamino-6-methylthio-s-triazin;
2-Chlor-4-ethylamino-6-(3-cyanopropylamino)-1,3,5-triazin;
4-Amino-6-t-butyl-3 -ethylthio-1,2,4-triazin-5(4H)-on;
N-Methyl-2-(1,3-benzthiazol-2-yloxy)-acetanilid;
N,N-Diisopropyl-S-(2,3,3-trichlorallyl)-thiolcarbamat;
2-Ethyl-6-methyl-N-(1-methyl-2-methoxyethyl)-chloracetanilid;
Chloressigsäure-N-(methoxymethyl)-2,6-diethylanilid;
2-Chlor-N-(2,6-dimethylphenyl)-N-[(1H)-pyrazol-1-yl-methyl]-acetamid;
α-Chlor-2',6'-diethyl-N-(2-propoxyethyl)-acetanilid;
2-[5-Methyl-5-(1-methylethyl)-4-oxo-2-imidazolin-2-yl]-3-chinolincarbonsäure;
Methyl-2-[4,5-dihydro-4-methyl-4-(1-methylethyl)-5-oxo-1H-imidazol-2-yl]-4(5)-methylbenzoat;
Methyl-5-(2,4-dichlorphenoxy)-2-nitrobenzoat;
5-(2-Chlor-4-trifluormethyl-phenoxy)-N-methylsulfonyl-2-nitrobenzamid;
(2-Ethoxy-1-methyl-2-oxo-ethyl)-5-[2-chlor-4-(trifluormethyl)-phenoxy]-2-nitrobenzoat;
5-(2-Chlor-4-trifluormethyl-phenoxy)-2-nitro-benzoesäure;
2,4-Dichlorphenoxyessigsäure;
2,4-Dichlorphenoxypropionsäure;
(2-Methyl-4-chlorphenoxy)-essigsäure;
(4-Chlor-2-methylphenoxy)-propionsäure;
3,5-Diiod-4-hydroxybenzonitril;
3,5-Dibrom-4-hydroxy-benzonitril;
2-Chlor-N-{[(4-methoxy-6-methyl-1,3,5-triazin-2-yl)-amino]-carbonyl}-benzolsulfonamid;
2-{[[((4-Methoxy-6-methyl-1,3,5-triazin-2-yl)-amino)-carbonyl]-amino]-sulfönyl}-benzoesäure oder deren Methylester 2-{-4-[(3-Chlor-5-(trifluormethyl)-2-pyridinyl)-oxy]-phenoxy}-propansäure bzw. -propansäureethylester;
2-[4-(3,5-Dichlorpyrid-2-yloxy)-phenoxy]-propionsäure-(trimethylsilylmethylester);
O-(6-Chlor-3-phenyl-pyridazin-4-yl)-S-octyl-thiocarbonat und 3-Isopropyl-2,1,3-benzothiadiazin-4-on-2,2-dioxid sind möglich. Einige Mischungen zeigen überraschenderweise auch synergistische Wirkung.

Auch eine Mischung mit anderen bekannten Wirkstoffen, wie Fungiziden, Insektiziden, Akariziden, Nematiziden, Schutzstoffen gegen Vogelfraß, Pflanzennährstoffen und Bodenstrukturverbesserungsmitteln ist möglich.

Die Wirkstoffe können als solche, in Form ihrer Formulierungen oder den daraus durch weiteres Verdünnen bereiteten Anwendungsformen, wie gebrauchsfertige Lösungen, Suspensionen, Emulsionen, Pulver, Pasten und Granulate angewandt werden. Die Anwendung geschieht in üblicher Weise, z.B. durch Gießen, Spritzen, Sprühen, Streuen.

Die erfindungsgemäßen Wirkstoffe können sowohl vor als auch nach dem Auflaufen der Pflanzen appliziert werden. Sie können auch vor der Saat in den Boden eingearbeitet werden.

Die angewandte Wirkstoffmenge kann in einem größeren Bereich schwanken. Sie hängt im wesentlichen von der Art des gewünschten Effektes ab. Im allgemeinen liegen die Aufwandmengen zwischen 0,01 g und 10 kg Wirkstoff pro Hektar Bodenfläche, vorzugsweise zwischen 0,05 g und 5 kg pro ha.

Bei der Anwendung als Wachstumsregulatoren können die erfindungsgemäßen Wirkstoffe in den Formulierungen ebenfalls in Mischung mit anderen bekannten Wirkstoffen vorliegen, wie Fungizide, Insektizide, Akarizide und Herbizide, sowie in Mischungen mit Düngemitteln und anderen Wachstumsregulatoren.

Die Wirkstoffe können dabei als solche, in Form ihrer Formulierungen oder den daraus bereiteten Anwendungsformen, wie gebrauchsfertige Lösungen, emulgierbare Konzentrate, Emulsionen, Schäume, Suspensionen, Spritzpulver, Pasten, lösliche Pulver, Stäubemittel und Granulate, angewendet werden. Die Anwendung geschieht in üblicher Weise, z.B. durch Gießen, Verspritzen, Versprühen, Verstreuen, Verstäuben, Verschäumen, Bestreichen usw. Es ist ferner möglich, die Wirkstoffe nach dem Ultra-Low-Volume-Verfahren auszubringen oder die Wirkstoffzubereitung oder den Wirkstoff selbst in den Boden zu injizieren. Es kann auch das Saatgut der Pflanzen behandelt werden.

Die Aufwandmengen können dabei ebenfalls in einem größeren Bereich variiert werden. Im allgemeinen verwendet man bei der Anwendung als Wachstumsregulatoren pro Hektar Bodenfläche 0,01 bis 50 kg, bevorzugt 0,05 bis 10 kg an Wirkstoff.

Für die Anwendungszeit gilt, daß die Anwendung der Wachstumsregulatoren in einem bevorzugten Zeitraum vorgenommen wird, dessen genaue Abgrenzung sich nach den klimatischen und vegetativen Gegebenheiten richtet.

### Herstellungsbeispiele

### Beispiel 1

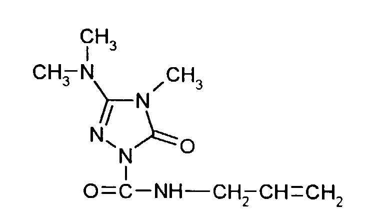

### (Verfahren a)

Zu 10,25 g (0,05 mol) 1-Chlorcarbonyl-3-dimethylamino-4-methyl-1,2,4-triazolin-5-on in 200 ml Acetonitril tropft man unter Rühren 5,7 g (0,1 mol) Allylamin so, daß die Reaktionstemperatur 40°C nicht übersteigt. Nach beendeter Zugabe rührt man vier Stunden bei Raumtemperatur, filtriert dann ausgefallenes Allylaminhydrochlorid ab, engt das Filtrat im Vakuum ein, nimmt den öligen Rückstand in 150 ml Dichlormethan auf, wäscht dreimal mit jeweils 50 ml Wasser, trocknet über Natriumsulfat und entfernt das Lösungsmittel im Vakuum.

Man erhält 8,8 g (79 % der Theorie) an 1-Allylaminocarbonyl-3-dimethylamino-4-methyl-1,2,4-triazolin-5-on als Öl.

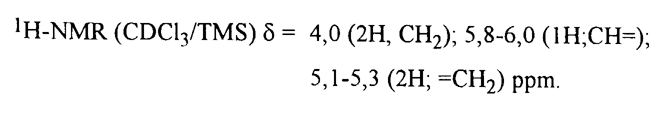

### Herstellung der Ausgangsverbindunzen

### Beispiel II-1

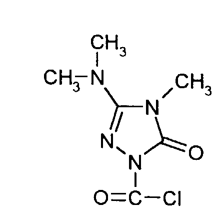

71 g (0,5 mol) 3-Dimethylamino-4-methyl-1H-1,2,4-triazolin-5-on in 300 ml Toluol werden unter Einleiten von Phosgen auf 120°C erwärmt. Insgesamt leitet man 115 g (1,15 mol) Phosgen ein. Ab 80°C findet eine lebhafte Chlorwasserstoffentwicklung statt. Nach beendeter Phosgen-Einleitung rührt man weitere 5 Stunden bei 120°C, entfernt überschüssiges Phosgen und Chlorwasserstoff durch Ausblasen mit Stickstoff und filtriert die Mischung bei 20°C. Das Filtrat wird mit 1 l Cyclohexan verrührt, das ausgefallene Produkt abgesaugt, mit Cyclohexan gewaschen und getrocknet.

Man erhält 70 g (69 % der Theorie) an 1-Chlorcarbonyl-3-dimethylamino-4-methyl-1,2,4-triazolin-5-on vom Schmelzpunkt 78°C bis 80°C.

### Beispiel IVa-1

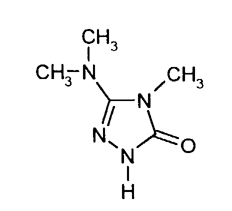

In eine Suspension aus 152,5 g (1mol) 1-Amino-2,2,3-trimethylguanidiniumhydrochlorid in 1 000 ml Acetonitril leitet man innerhalb von 2 Stunden unter Rühren bei 80°C 150 g (1,5 mol) Phosgen, rührt 30 Minuten bei 80°C nach, kühlt auf 20°C ab, entfernt überschüssiges Phosgen durch Ausblasen mit Stickstoff, saugt ausgefallenes Produkt ab, löst es in 1 000 ml Wasser, neutralisiert mit konzentrierter Natronlauge und engt im Vakuum zur Trockne ein. Der ölige Rückstand wird in 1 000 ml Acetonitril aufgenommen, filtriert und das Filtrat im Vakuum vom Lösungsmittel befreit.

Man erhält 80 g (57 % der Theorie) an 3-Dimethylamino-4-methyl-1H-1,2,4-triazolin-5-on vom Schmelzpunkt 78°C bis 80°C.

### Beispiel VII-1

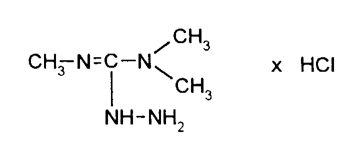

Zu 50 g (1 mol) Hydrazinhydrat in 300 ml Isopropanol tropft man bei 20°C bis 25°C unter Rühren innerhalb von 30 Minuten eine Lösung von 78,5 g (0,5 mol) Chlortrimethylformamidiniumhydrochlorid in 250 ml Isopropanol, rührt nach beendeter Zugabe weitere 30 Minuten bei Raumtemperatur, saugt ausgefallenes Hydrazinhydrochlorid ab, wäscht mit 150 ml Isopropanol nach und engt das Isopropanolfiltrat im Vakuum ein.

Man erhält 70,7 g (93 % der Theorie) an 1-Amino-2,2,3-trimethylguanidiniumhydrochlorid, welches ohne Reinigung weiter umgesetzt wird.

### Beispiel IX-1

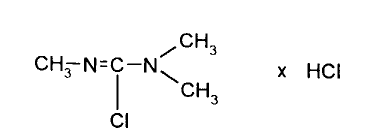

In eine Mischung aus 510 g (5 mol) N,N,N'-Trimethylharnstoff und 3 l Chlorbenzol leitet man bei 80°C innerhalb 2,5 Stunden unter Rühren 545 g (5,5 mol) Phosgen und rührt nach beendetem Einleiten weitere 45 Minuten bis zum Ende der Kohlendioxid-Entwicklung bei 80°C nach. Die Reaktionsmischung wird abgekühlt auf 10°C, das wasserempfindliche Produkt unter Stickstoff abgesaugt, mit 1 l Chlorbenzol und zweimal mit jeweils 500 ml Petrolether gewaschen und im Vakuum getrocknet.

Man erhält 635,3 g (81 % der Theorie) an Chlortrimethylformamidiniumhydrochlorid vom Schmelzpunkt 76°C bis 78°C.

### Beispiel 2

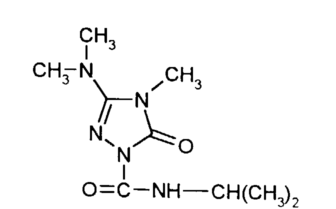

### (Verfahren b)

7,1 g (0,05 mol) 3-Dimethylamino-4-methyl-1H-1,2,4-triazolin-5-on in 100 ml Toluol werden mit 4,25 g (0,05 mol) Isopropylisocyanat versetzt und 2 Stunden bei 120°C gerührt. Das erkaltete Reaktionsgemisch wird filtriert und das Filtrat im Vakuum eingeengt.

Man erhält 9,8 g (87 % der Theorie) an 1-Isopropylaminocarbonyl-3-dimethylamino-4-methyl-1,2,4-triazolin-5-on vom Schmelzpunkt 36°C bis 38°C.

### Beispiel 3

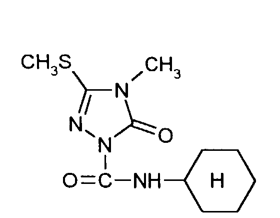

### (Verfahren b)

Zu 1,5 g (0,01 mol) 3-Methylthio-4-methyl-1H-1,2,4-triazolin-5-on (vgl. US-PS 4 098 896 bzw. US-PS 4 110 332) in 20 ml Dioxan gibt man 1 g (0,01 mol) Triethylamin und 1,3 g (0,01 mol) Cyclohexylisocyanat, rührt 12 Stunden bei 60°C, engt im Vakuum zur Trockne ein, nimmt den Rückstand in 50 ml Dichlormethan auf, filtriert, wäscht das Filtrat zweimal mit jeweils 50 ml Wasser, trocknet über Natriumsulfat, entfernt das Lösungsmittel im Vakuum und verreibt den Rückstand mit Ether.

Man erhält 2,2 g (81 % der Theorie) an 1-Cyclohexylaminocarbonyl-3-methylthio-4-methyl-1,2,4-triazolin-5-on vom Schmelzpunkt 136°C.

In entsprechender Weise und gemäß den allgemeinen Angaben zur Herstellung erhält man die folgenden substituierten Triazolinone der allgemeinen Formel (I):

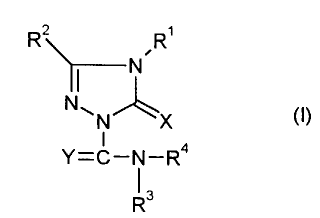

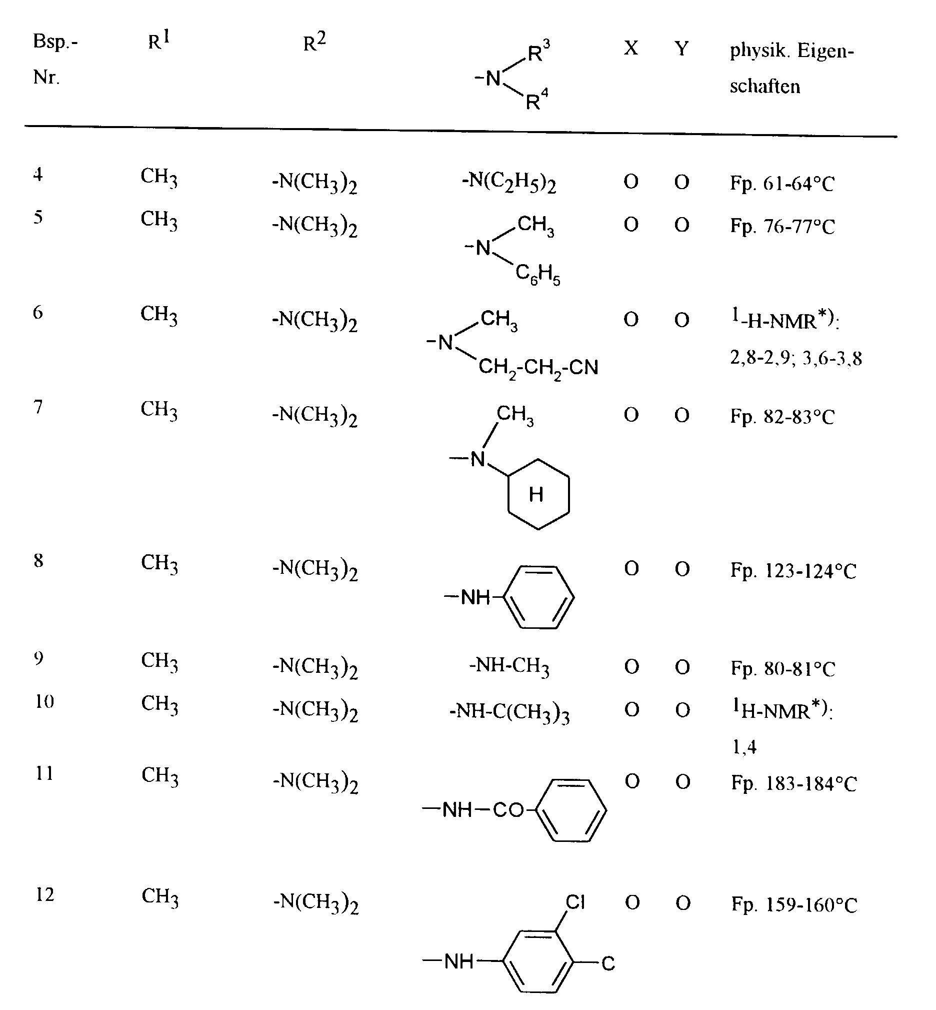

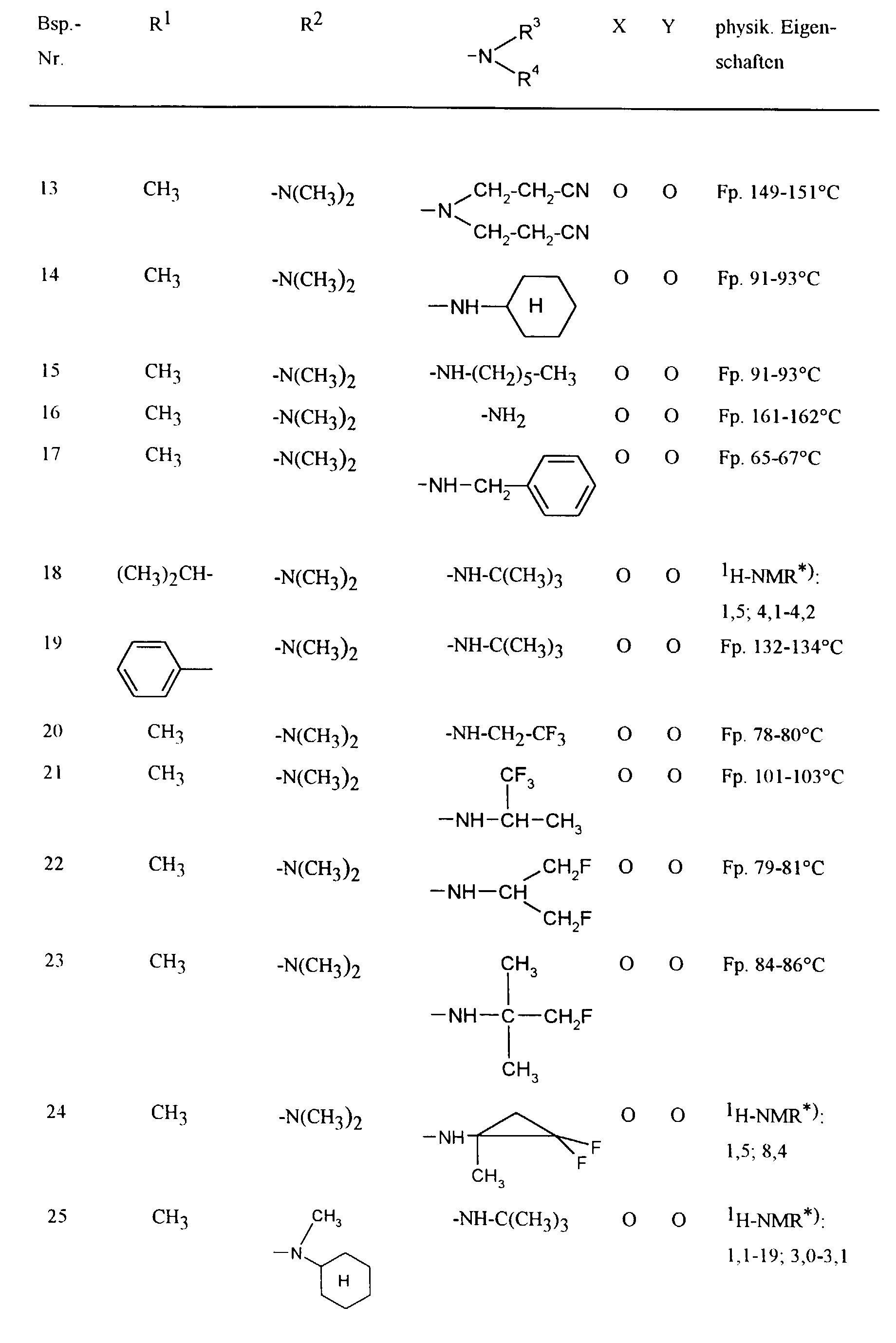

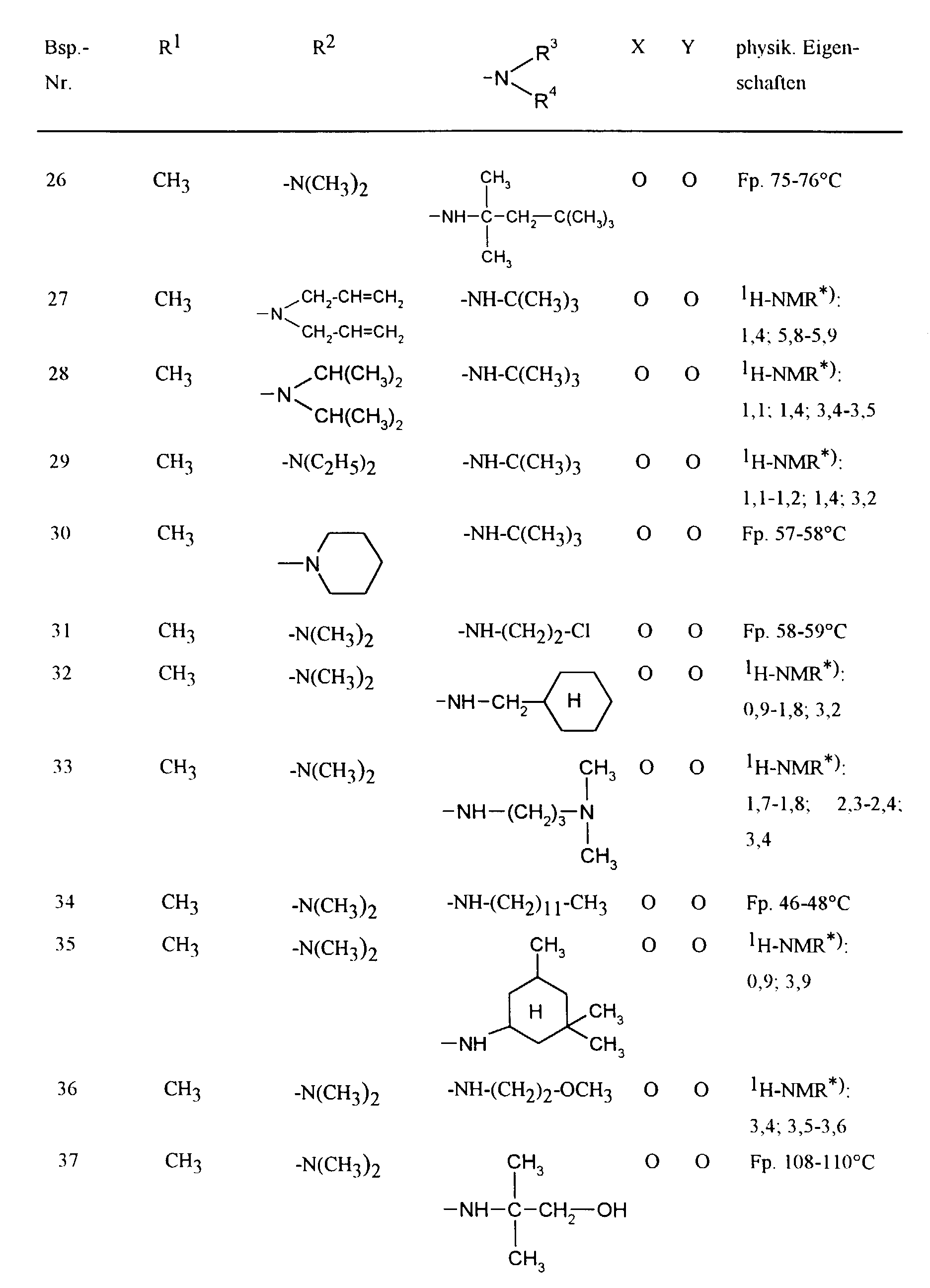

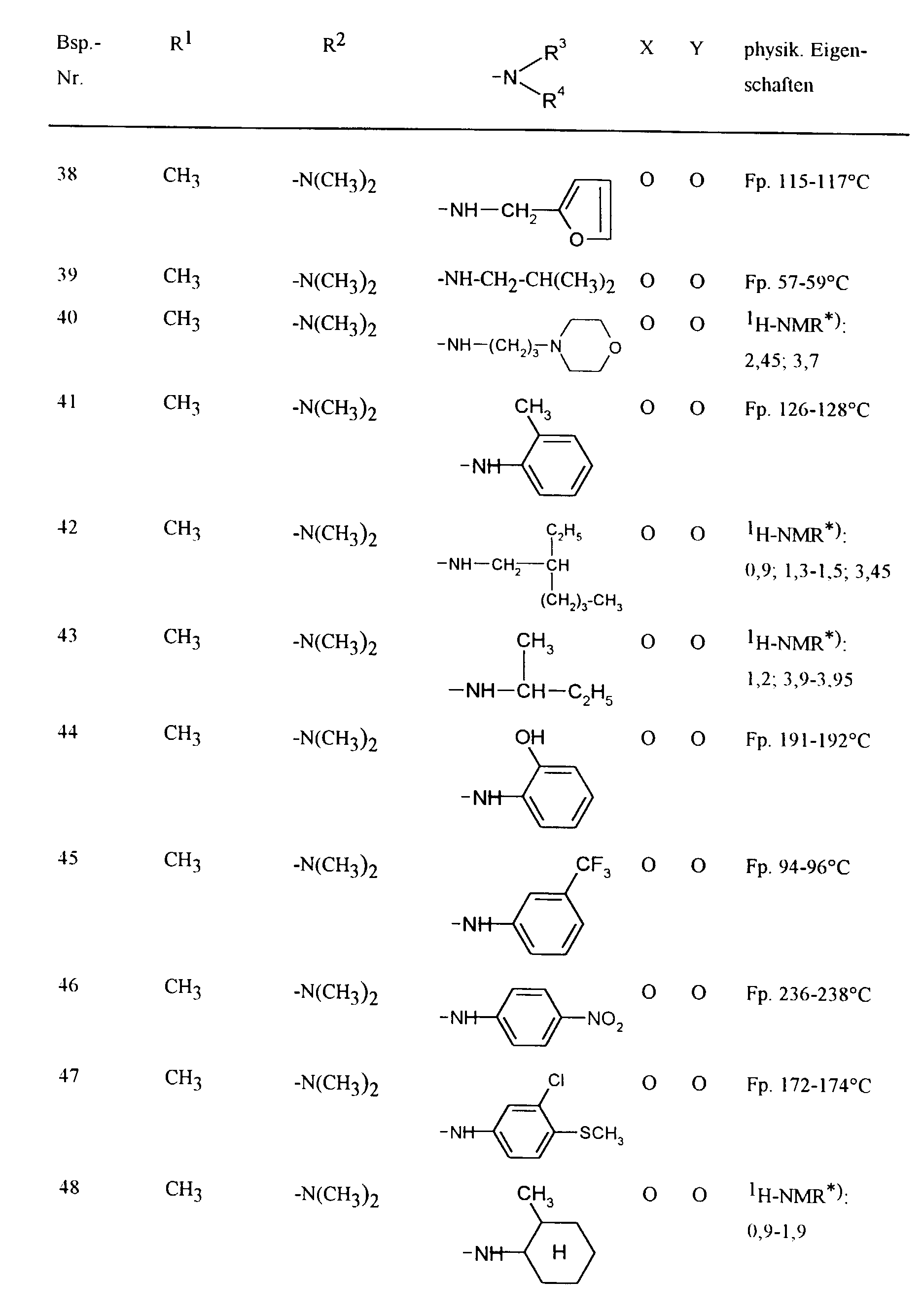

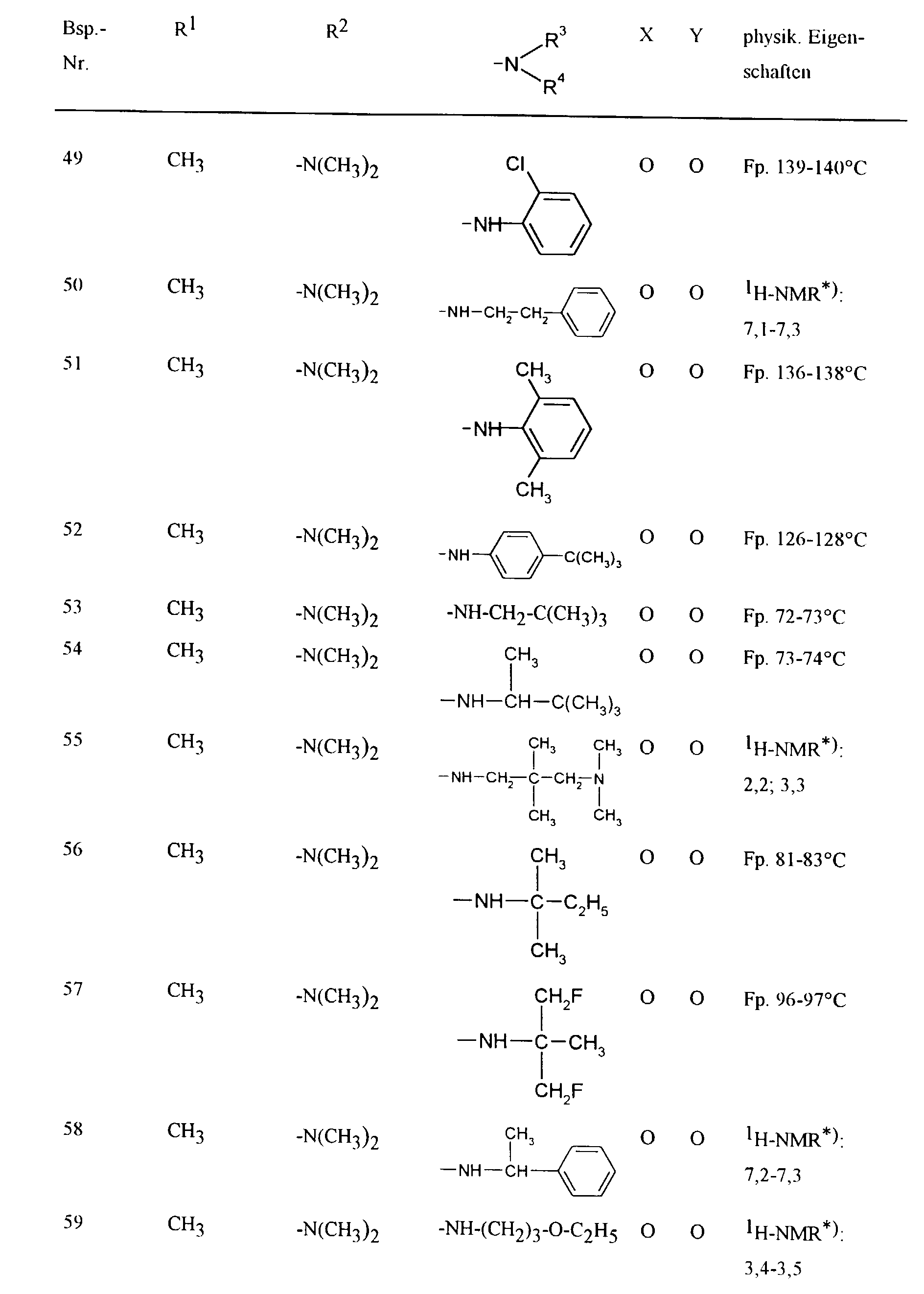

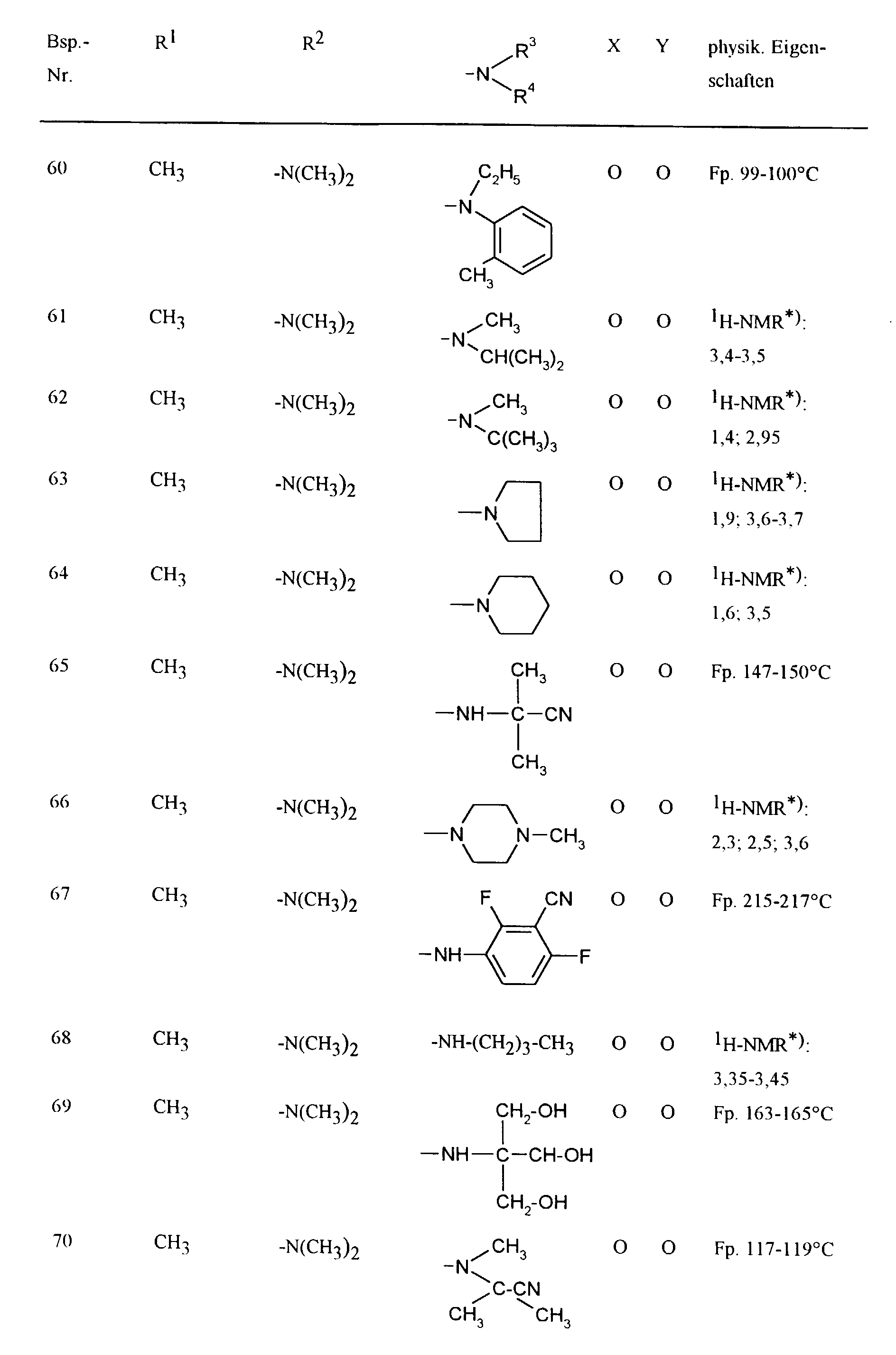

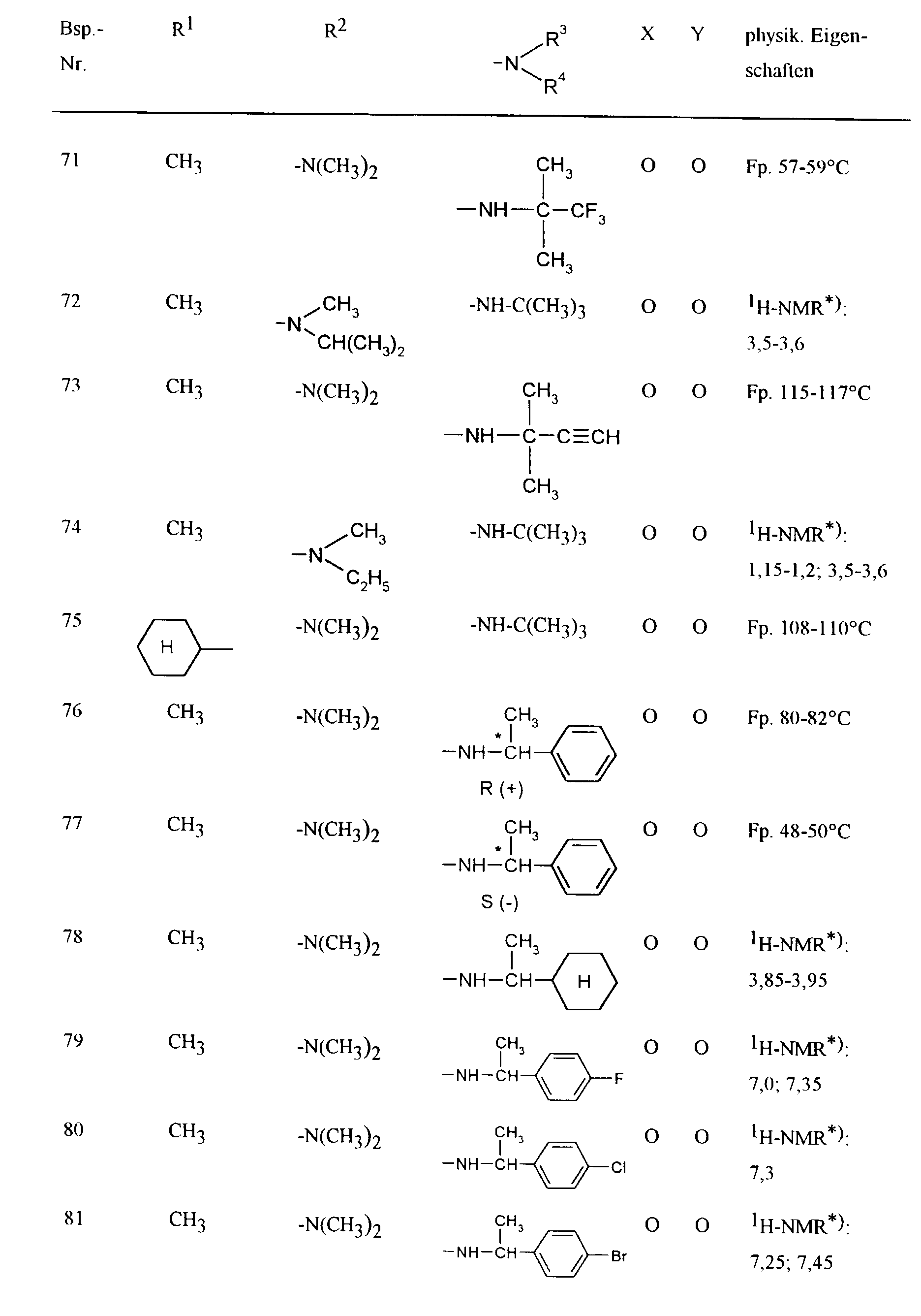

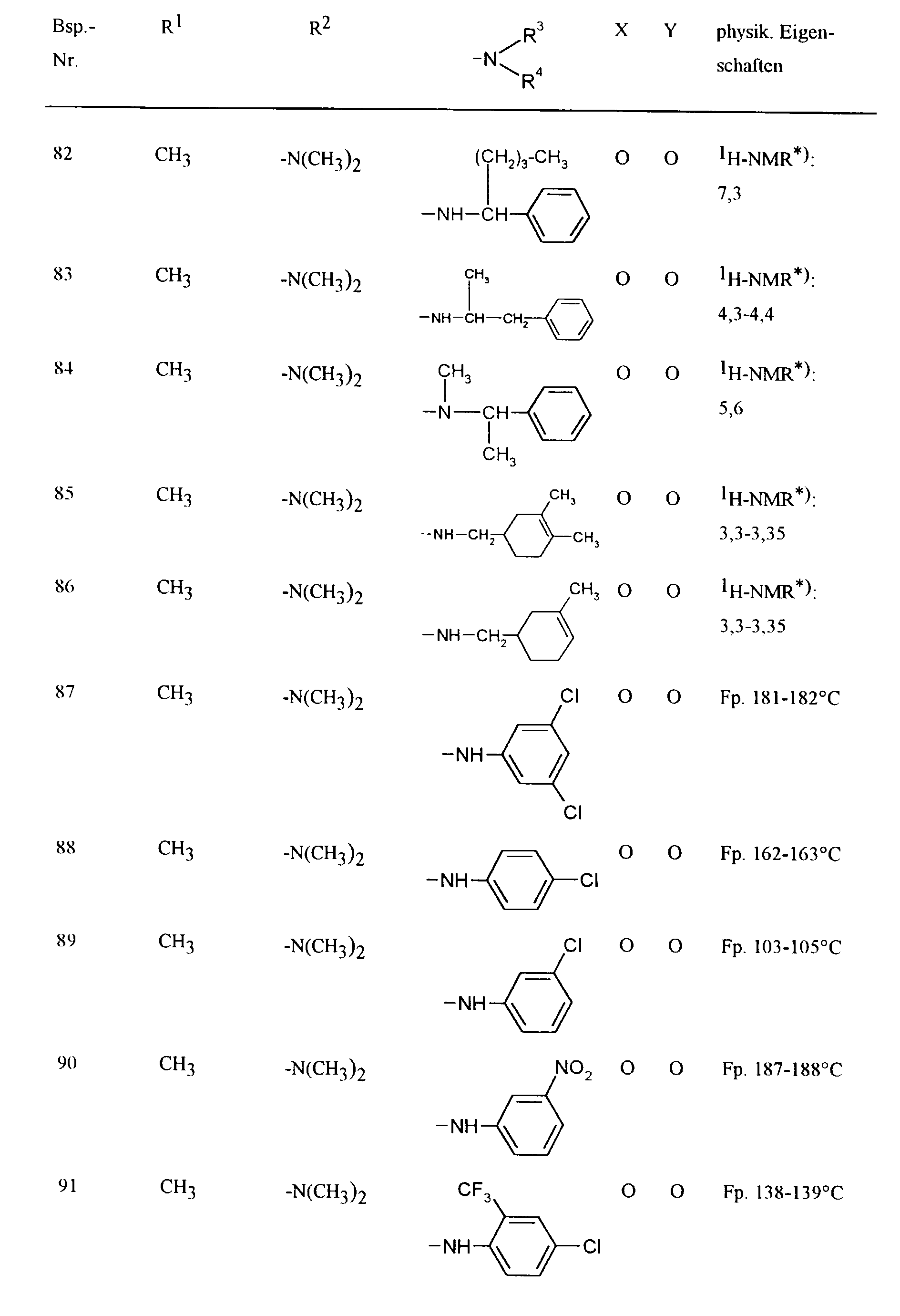

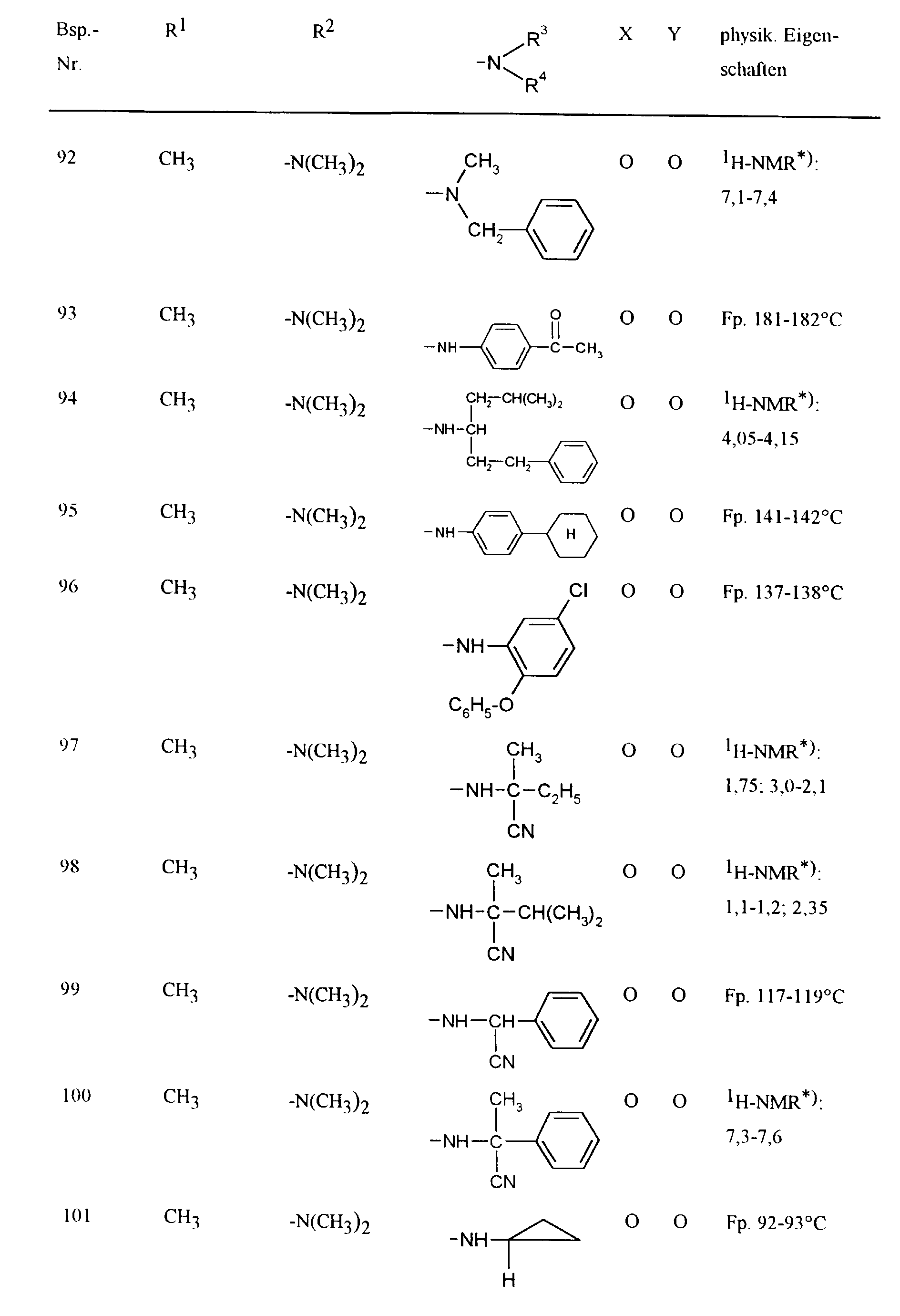

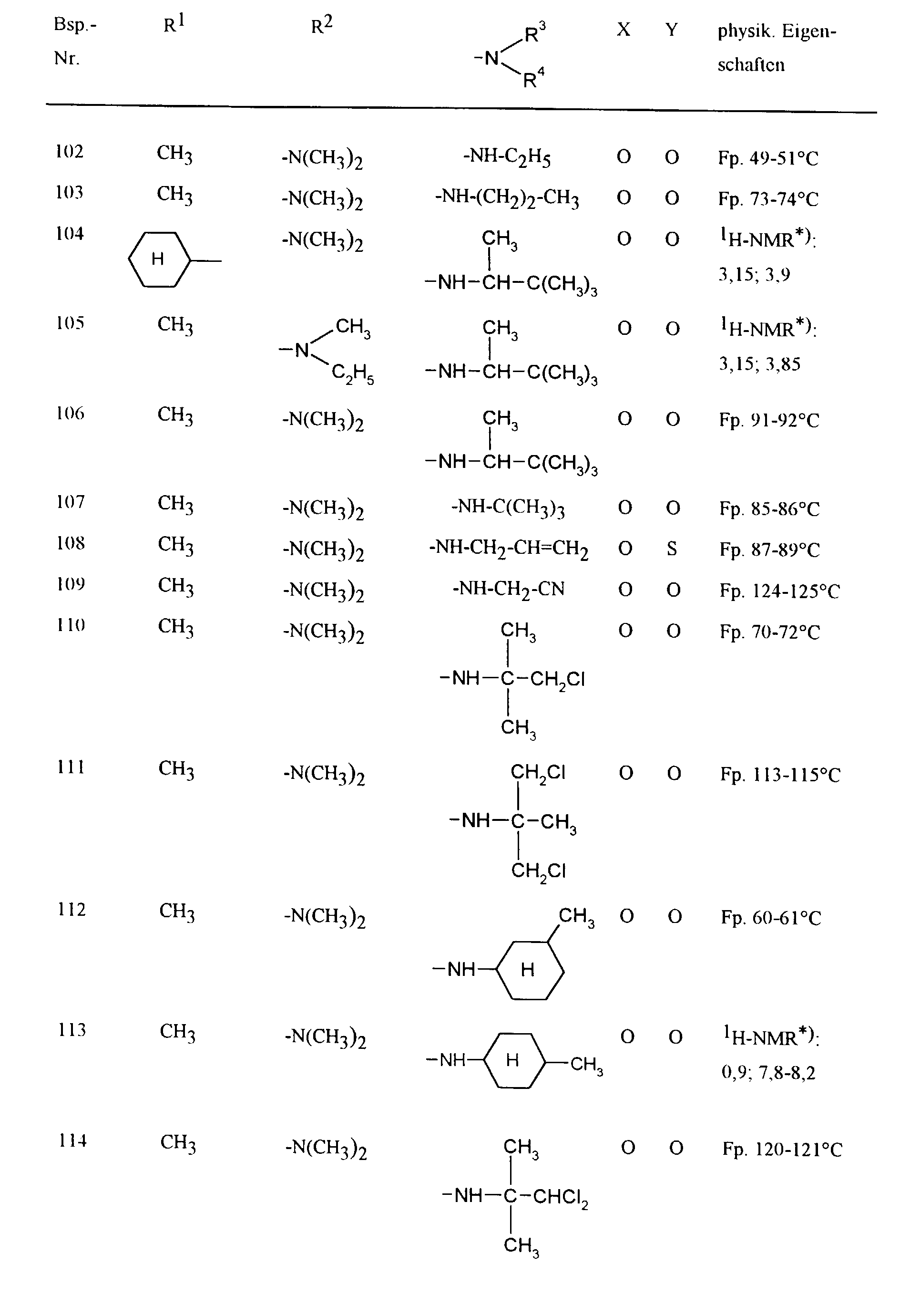

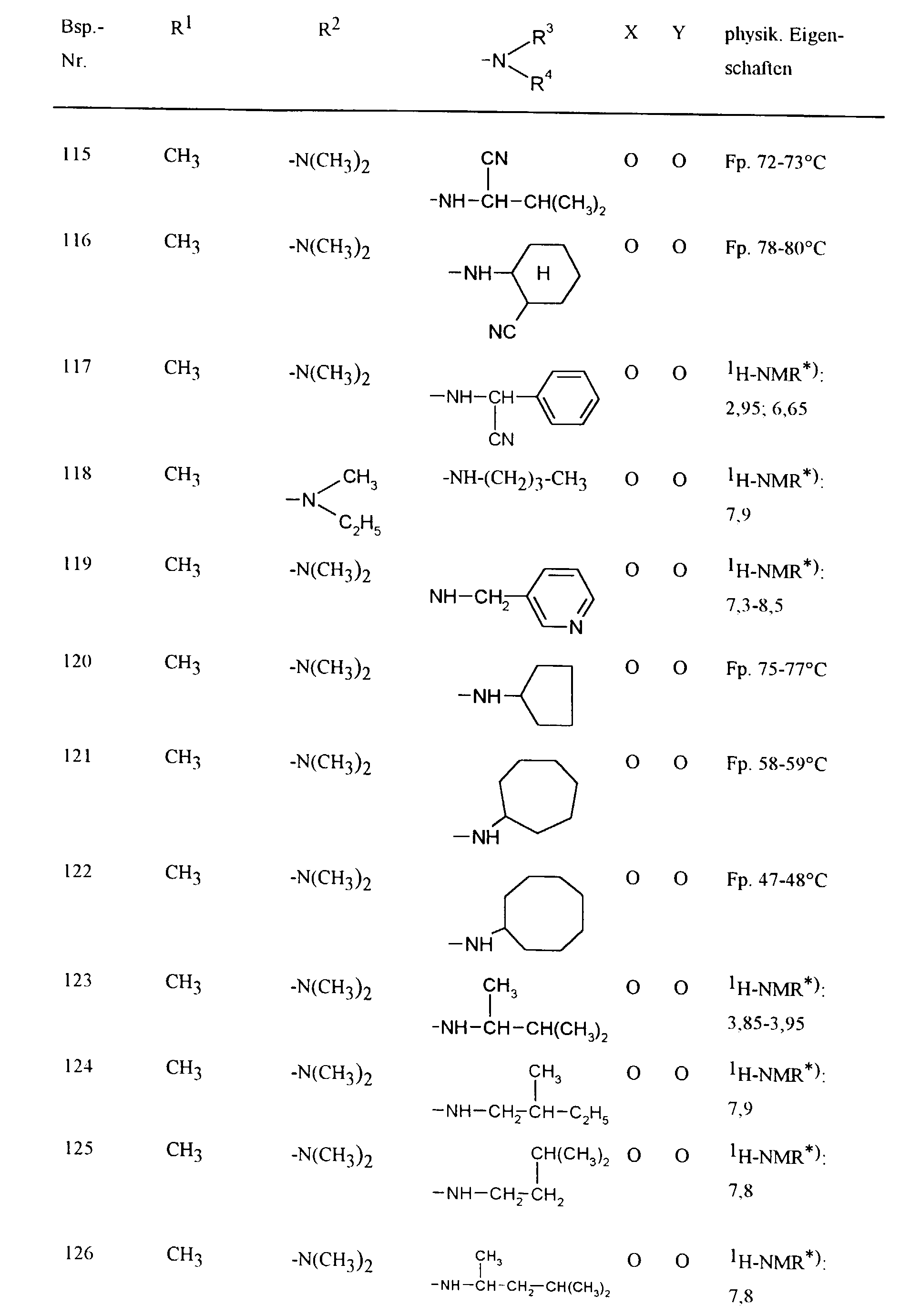

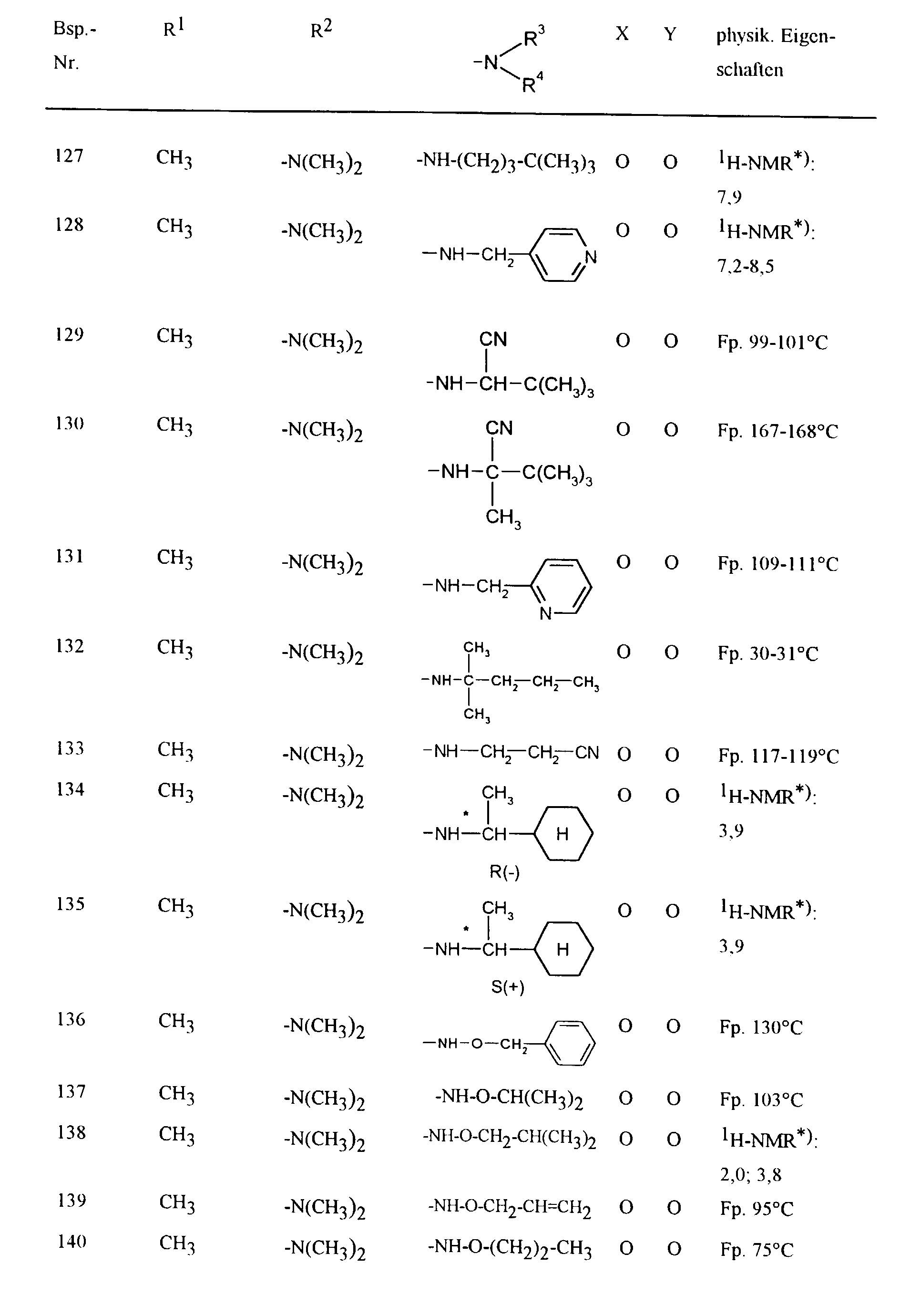

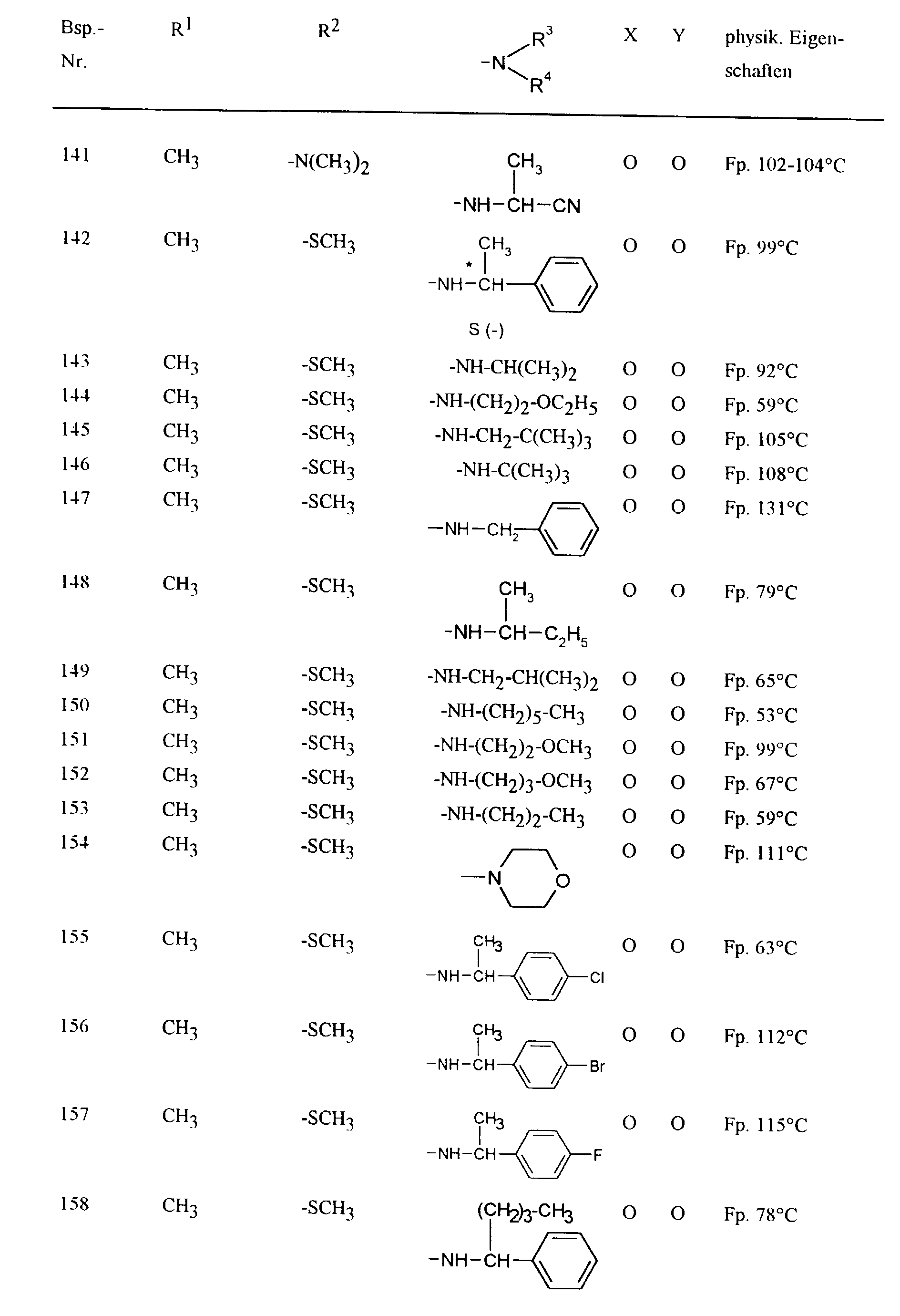

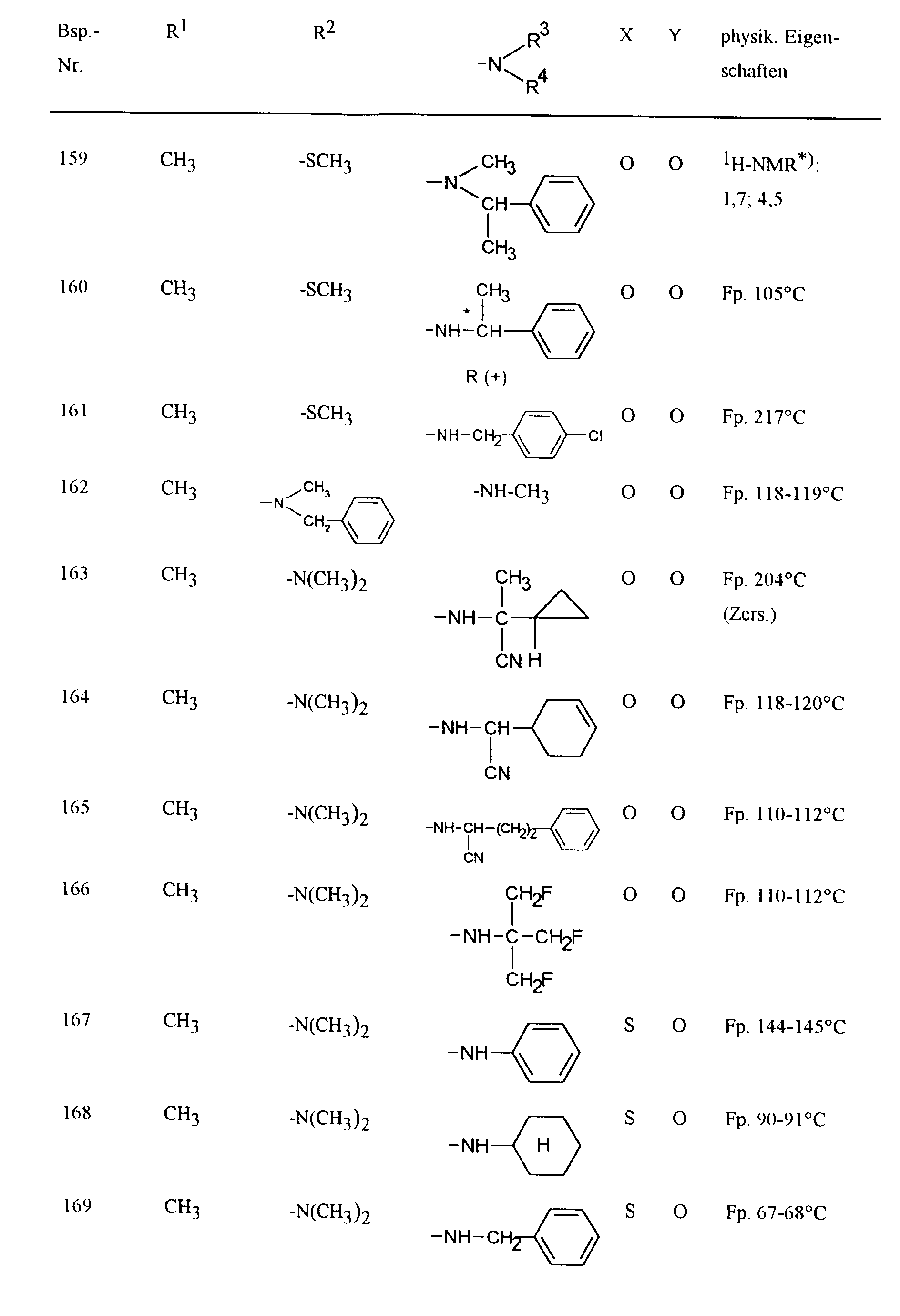

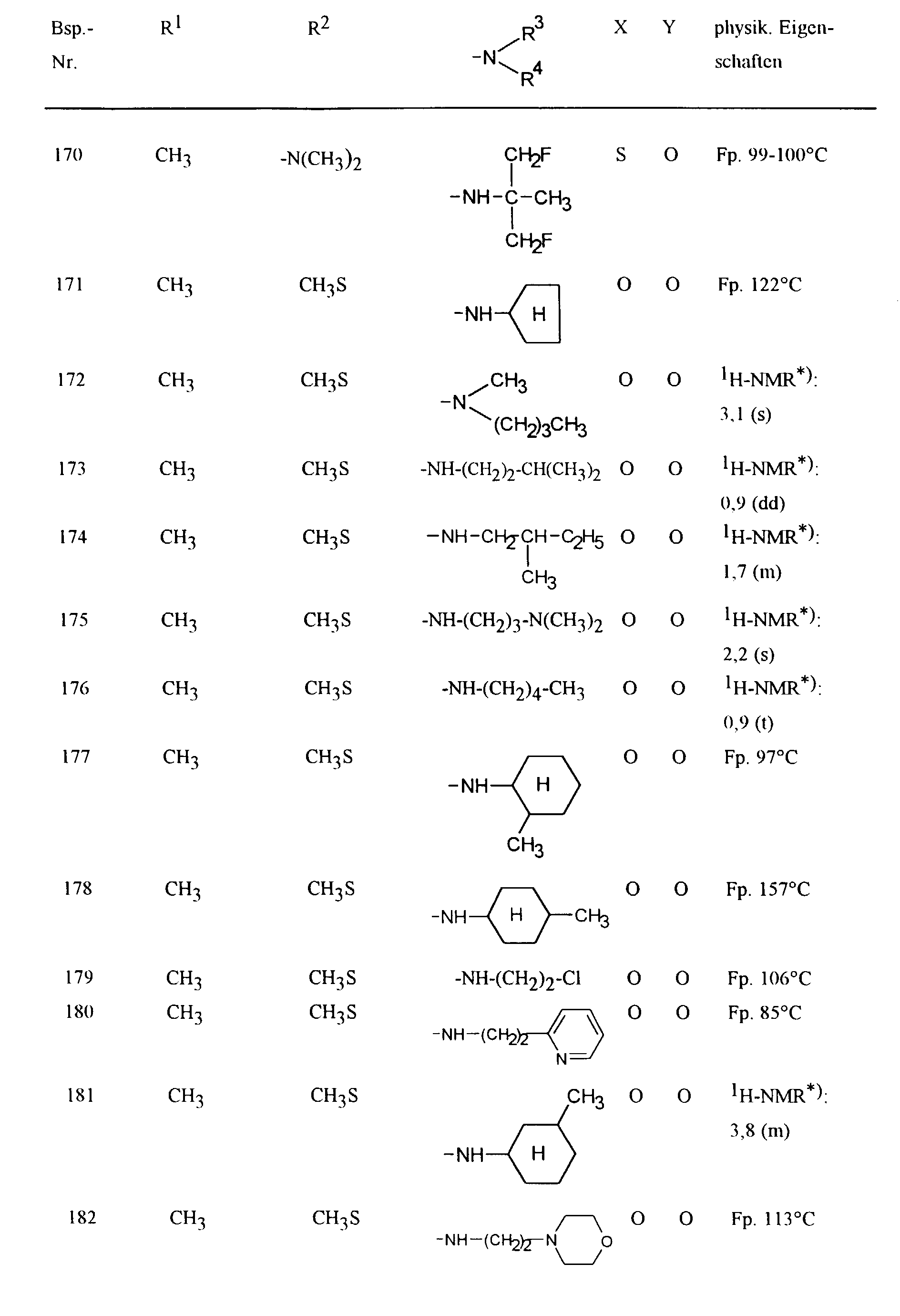

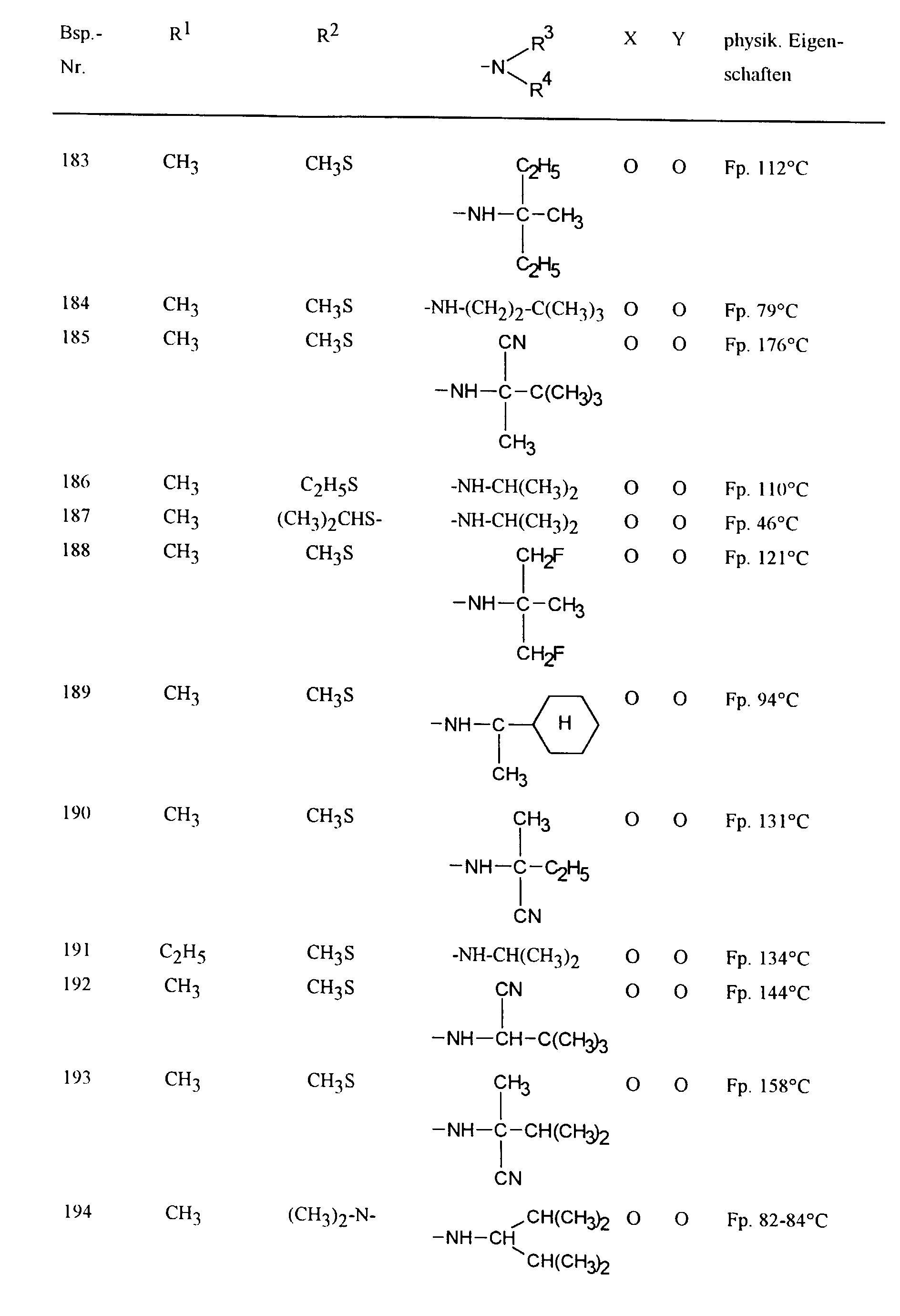

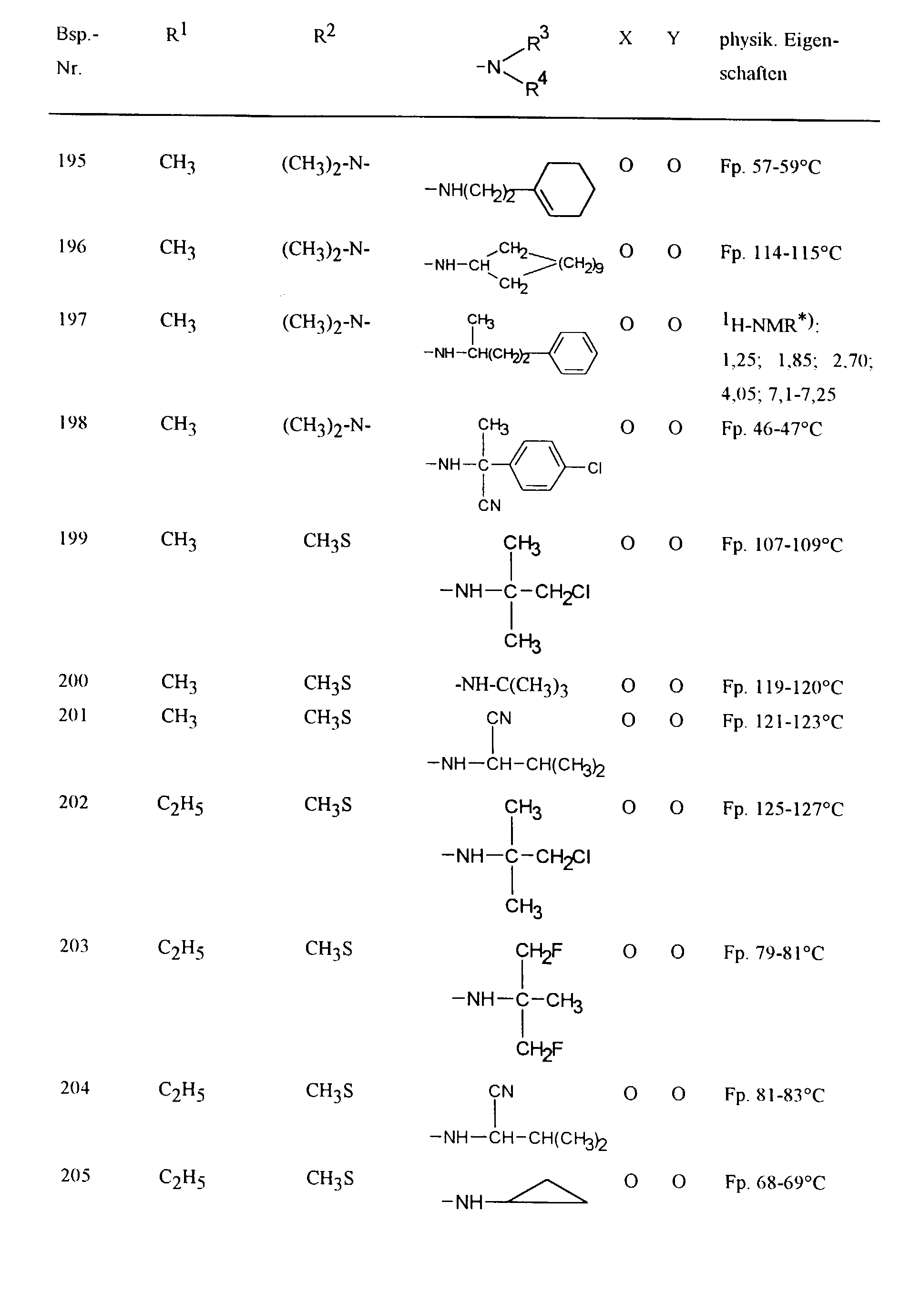

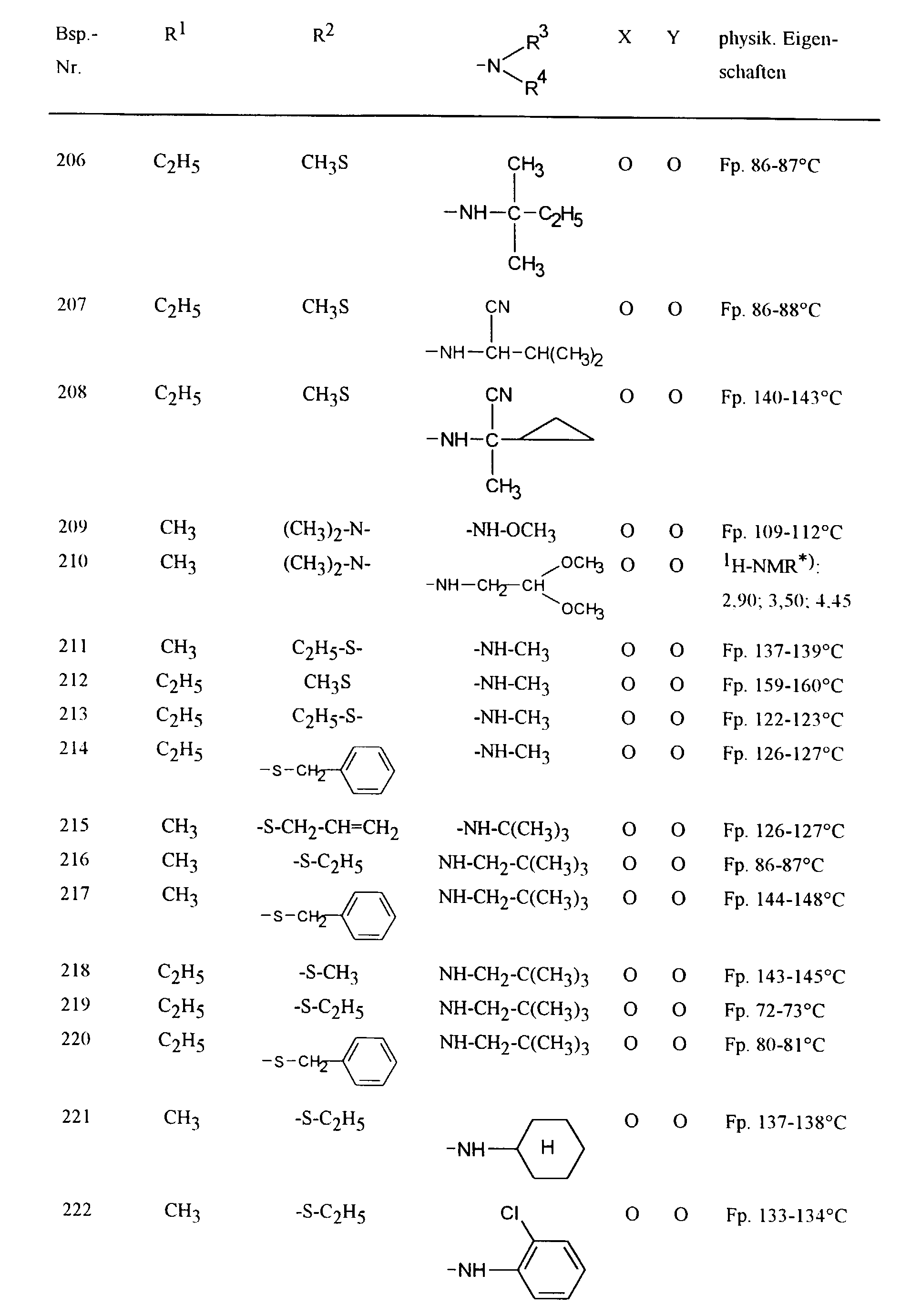

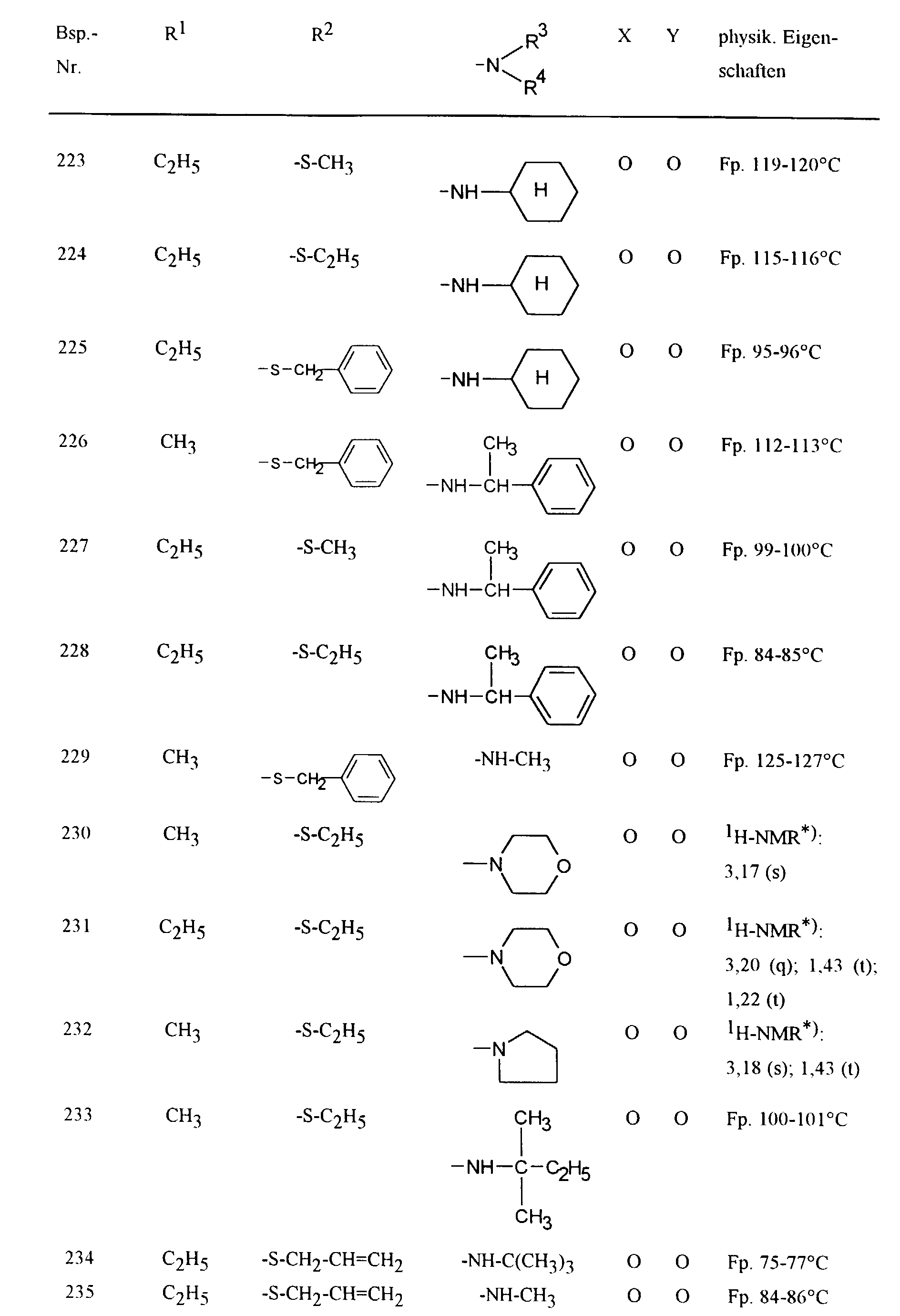

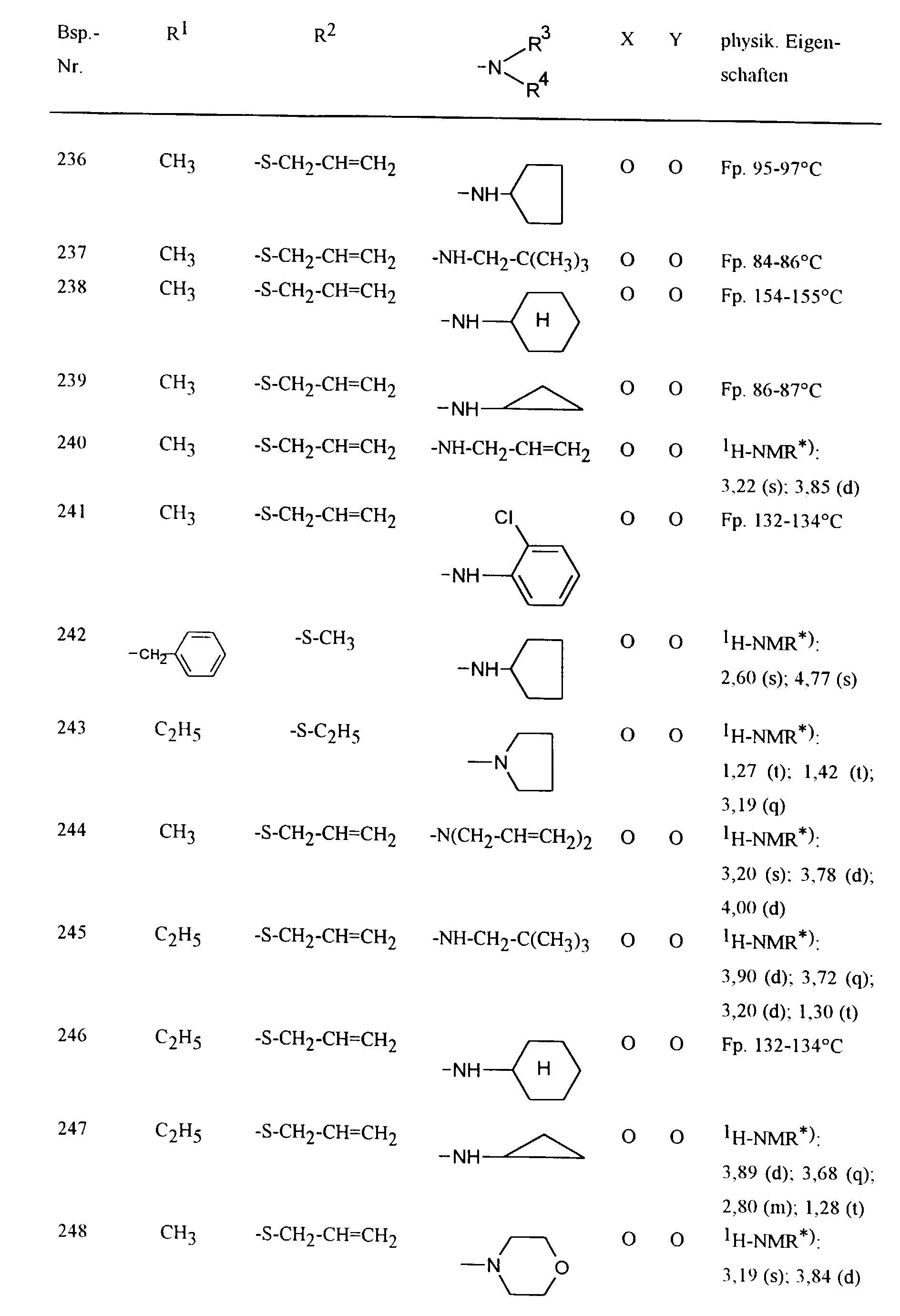

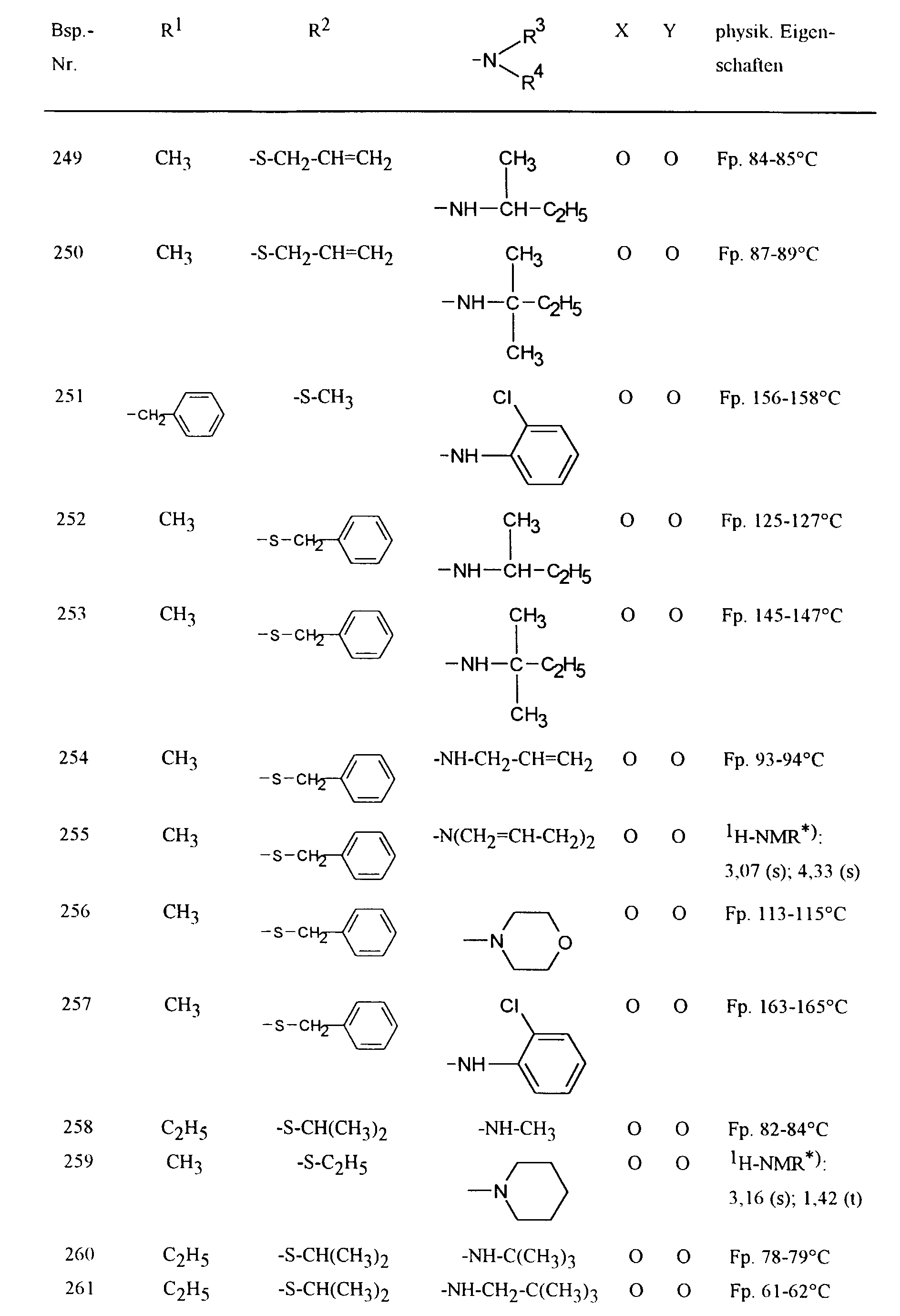

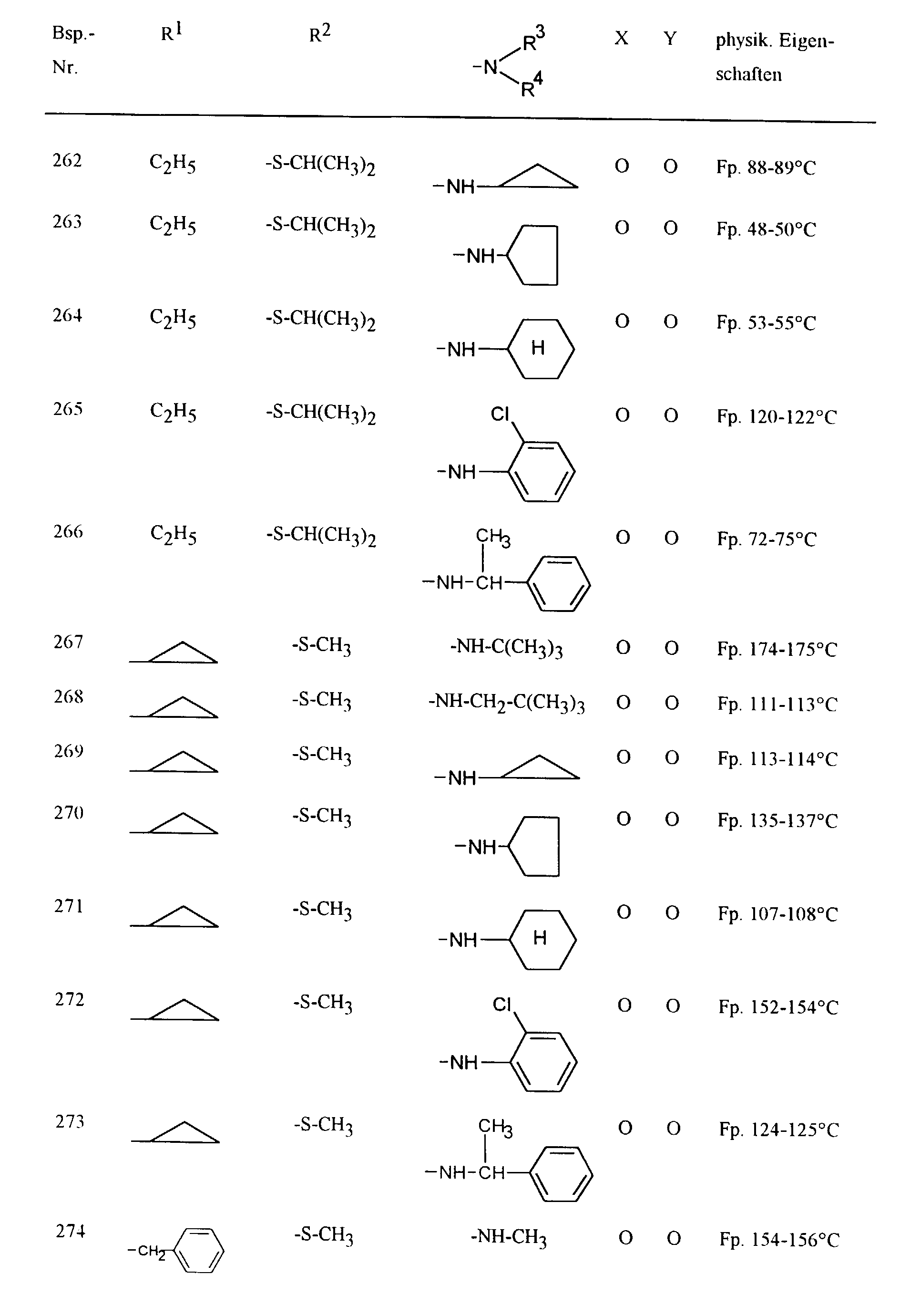

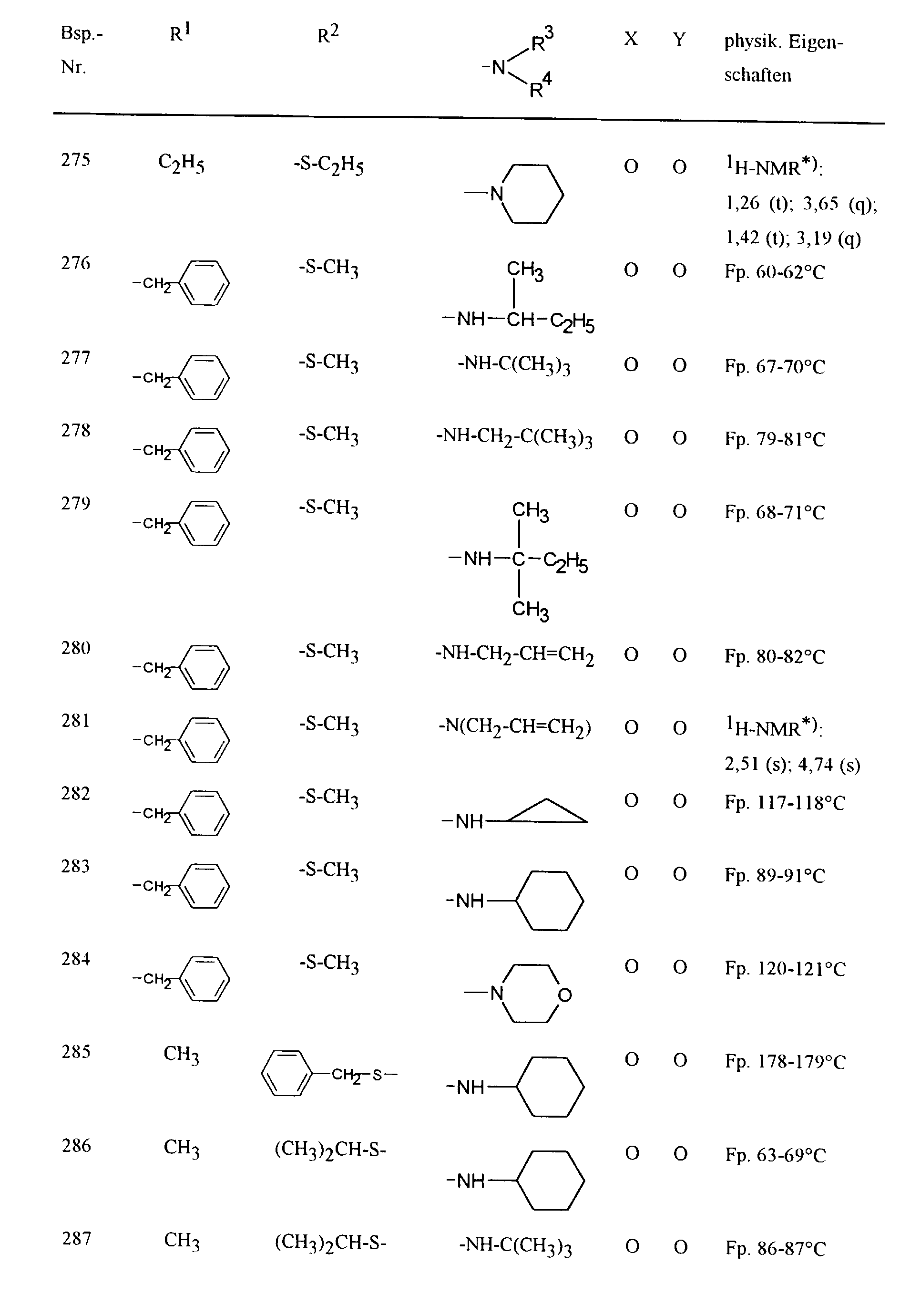

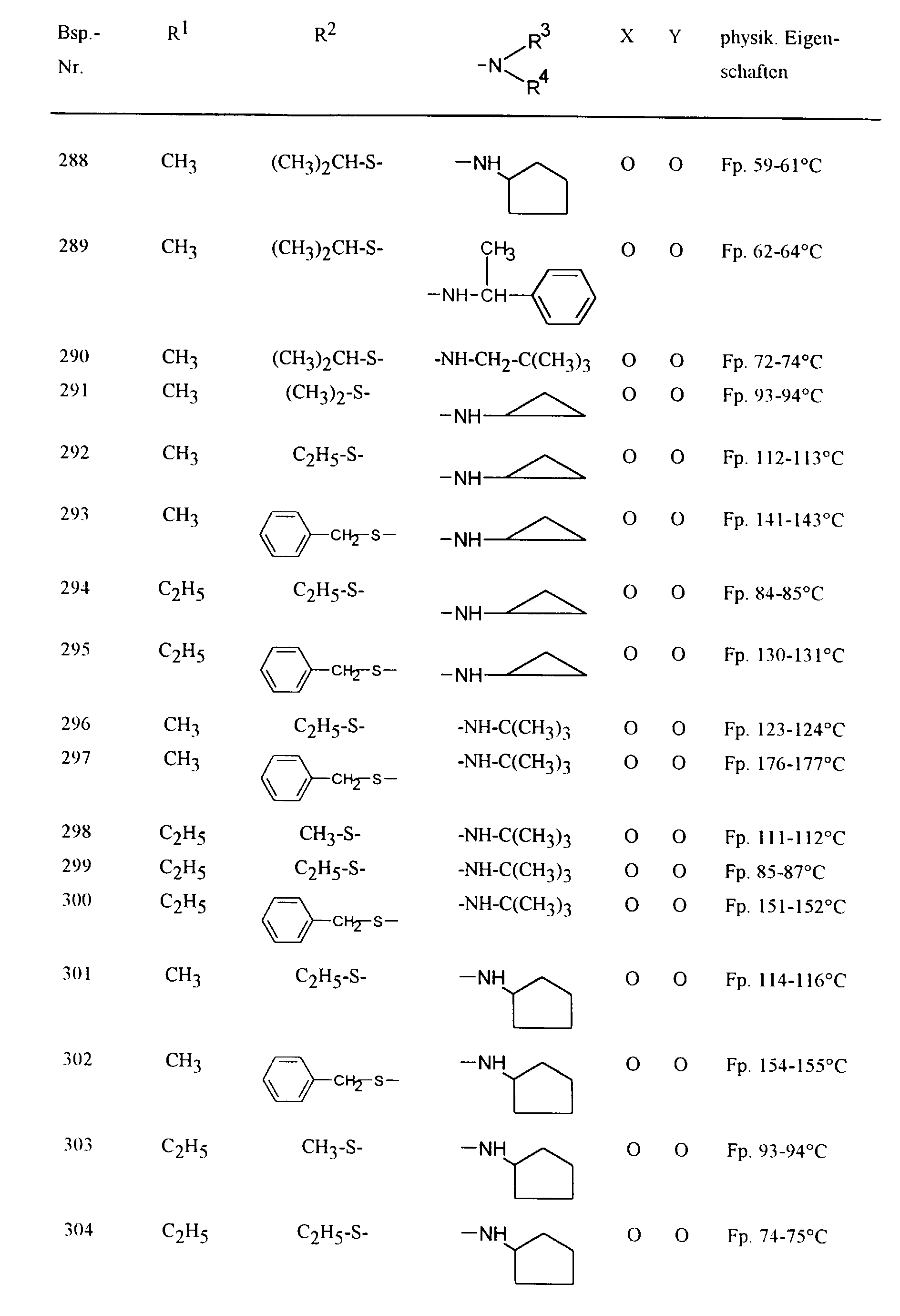

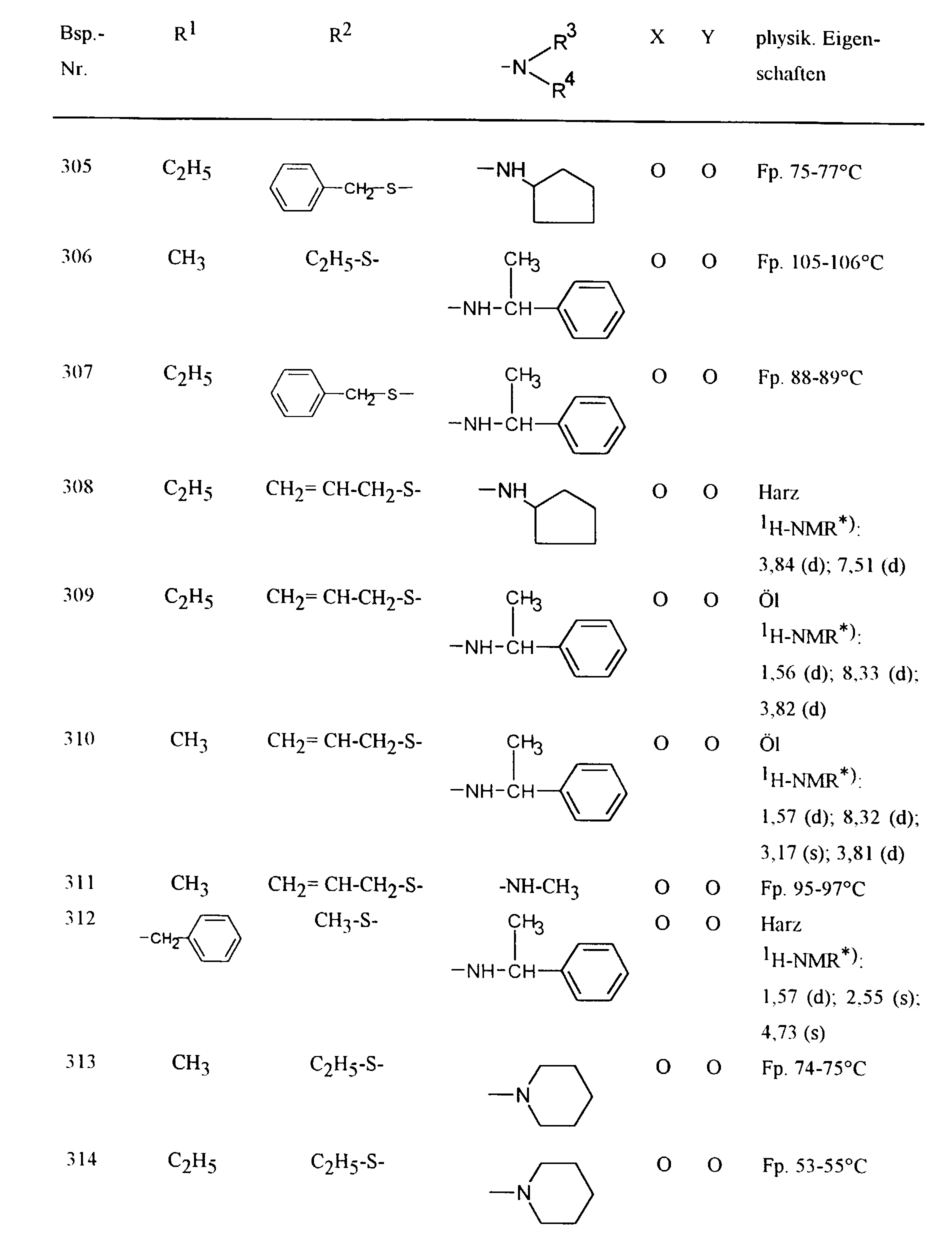

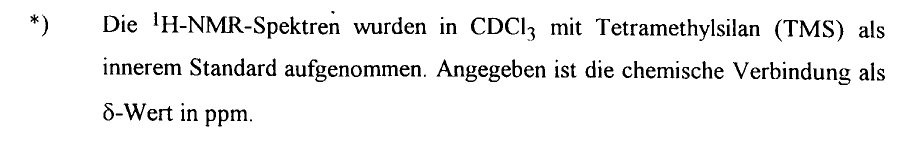

### Anwendungsbeispiele

In den folgenden Anwendungsbeispielen wurden die nachstehend aufgeführten Verbindungen als Vergleichssubstanzen eingesetzt: 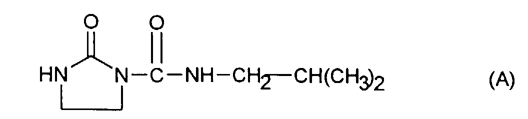 Imidazolidin-2-on-1-carbonsäureisobutylamid
(bekannt aus K.H. Büchel, ,,Pflanzenschutz und Schädlingsbekämpfung" S. 170, Thieme Verlag Stuttgart 1977)

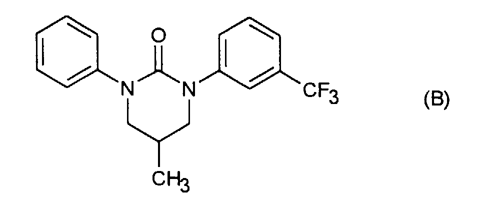

1-Phenyl-3-(3-trifluormethylphenyl)-5-methylperhydropynmidin-2-on
(bekannt aus EP 58 868)

### Beispiel A

### Pre-emergence-Test

- Lösungsmittel:: 5 Gewichtsteile Aceton
- Emulgator:: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Samen der Testpflanzen werden in normalen Boden ausgesät und nach 24 Stunden mit der Wirkstoffzubereitung begossen. Dabei hält man die Wassermenge pro Flächeneinheit zweckmäßigerweise konstant. Die Wirkstoffkonzentration in der Zubereitung spielt keine Rolle, entscheidend ist nur die Aufwandmenge des Wirkstoffs pro Flächeneinheit. Nach 3 Wochen wird der Schädigungsgrad der Pflanzen bonitiert in % Schädigung im Vergleich zur Entwicklung der unbehandelten Kontrolle. Es bedeuten:
0 % = keine Wirkung (wie unbehandelte Kontrolle)
100 % = totale Vernichtung

Eine deutliche Überlegenheit in der Wirksamkeit gegenüber dem Stand der Technik zeigen in diesem Test z.B. die Verbindungen gemäß der Herstellungsbeispiele 2, 10, 14, 39, 43, 48, 53, 54, 56, 57, 58, 65, 68 und 71.

### Beispiel B

Post-emergence-Test
- Lösungsmittel:: 5 Gewichtsteile Aceton
- Emulgator:: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Mit der Wirkstoffzubereitung spritzt man Testpflanzen, welche eine Höhe von 5 bis 15 cm haben so, daß die jeweils gewünschten Wirkstoffmengen pro Flächeneinheit ausgebracht werden. Die Konzentration der Spritzbrühe wird so gewählt, daß in 2000 l Wasser/ha die jeweils gewünschten Wirkstoffmengen ausgebracht werden. Nach 3 Wochen wird der Schädigungsgrad der Pflanzen bonitiert in % Schädigung im Vergleich zur Entwicklung der unbehandelten Kontrolle. Es bedeuten:
0 % = keine Wirkung (wie unbehandelte Kontrolle)
100 % = totale Vernichtung

Eine deutliche Überlegenheit in der Wirksamkeit gegenüber dem Stand der Technik zeigen in diesem Test z.B. die Verbindungen gemäß der Herstellungsbeispiele 1, 2, 10, 14, 17, 32, 34, 36, 37, 39, 43, 50, 53, 54, 56, 57, 58, 65 und 68.

### Beispiel C

Entlaubung und Austrocknung der Blätter bei Baumwolle
- Lösungsmittel:: 30 Gewichtsteile Dimethylformamid
- Emulgator:: 1 Gewichtsteil Polyoxyethylen-Sorbitan-Monolaurat

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Baumwollpflanzen werden im Gewächshaus bis zur vollen Entfaltung des 5. Folgeblattes angezogen. In diesem Stadium werden die Pflanzen tropfnaß mit den Wirkstoffzubereitungen besprüht. Nach 1 Woche werden der Blattfall und das Austrocknen der Blätter im Vergleich zu den Kontrollpflanzen bonitiert. Es bedeuten:
- 0: kein Austrocknen der Blätter, kein Blattfall
- +: leichtes Austrocknen der Blätter, geringer Blattfall
- ++: starkes Austrocknen der Blätter, starker Blattfall
- +++: sehr starkes Austrocknen der Blätter, sehr starker Blattfall

Eine deutliche Überlegenheit in der Wirksamkeit im Vergleich zur unbehandelten Kontrolle zeigen in diesem Test beispielsweise die Verbindungen gemäß der Herstellungsbeispiele 15, 17, 32, 43, 48, 54, 56, 57 und 58.

## Patentansprüche

1. Substituierte Triazolinone der allgemeinen Formel (IVa) 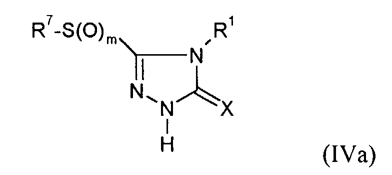 in welcher
R¹ für Methoxy, Ethoxy, Methoxymethyl, für geradkettiges oder verzweigtes Halogenalkyl mit 1 bis 4 Kohlenstoffatomen und 1 bis 9 gleichen oder verscheidenen Halogenatomen, insbesondere Fluor, Chlor oder Brom, für Cyclopropylmethyl, Cyclohexylmethyl, Cyclohexylethyl steht,
X für Sauerstoff steht,
R⁷ für Methyl, Ethyl, n- oder i-Propyl, n-, i-, s- oder t-Butyl, Allyl, Propargyl, Cyclopentyl, Cyclohexyl, Cyclohexylmethyl, Cyclohexylethyl steht und
m für die Zahl 0 steht.

2. Substituiertes Triazolinon der Formel 4-Cyclopropyl-2,4-dihydro-5-methylthio-3H-1,2,4-triazol-3-on
